(19) Europäisches Patentamt
European Patent Office
Office européen des brevets

(11) Veröffentlichungsnummer: **0 411 420 A2**

(12) # EUROPÄISCHE PATENTANMELDUNG

(21) Anmeldenummer: **90114015.2**

(22) Anmeldetag: **21.07.90**

(51) Int. Cl.[5]: **C07D 213/53**, C07D 401/06, A61K 31/44

(30) Priorität: **03.08.89 DE 3925636**

(43) Veröffentlichungstag der Anmeldung: **06.02.91 Patentblatt 91/06**

(84) Benannte Vertragsstaaten: **AT BE CH DE DK ES FR GB GR IT LI LU NL SE**

(71) Anmelder: **BAYER AG**

**D-5090 Leverkusen 1 Bayerwerk(DE)**

(72) Erfinder: **Angerbauer, Rolf, Dr.**
**Am Moospfad 20**
**D-5600 Wuppertal 1(DE)**
Erfinder: **Fey, Peter, Dr.**
**Ursulastrasse 14**
**D-5600 Wuppertal 1(DE)**
Erfinder: **Hübsch, Walter, Dr.**
**Am Eckbusch 43/50**
**D-5600 Wuppertal 1(DE)**
Erfinder: **Philipps, Thomas, Dr.**
**Silesiusstrasse 74**
**D-5000 Köln 80(DE)**
Erfinder: **Bischoff, Himlar, Dr.**
**Am Rohm 78**
**D-5600 Wuppertal 1(DE)**
Erfinder: **Petzinna, Dieter, Dr.**
**Peter-Berten-Strasse 10**
**D-4000 Düsselsorf 12(DE)**
Erfinder: **Schmidt, Delf, Dr.**
**Am Eckbusch 55b**
**D-5600 Wuppertal 1(DE)**

(54) **Imino-substituierte Pyridine.**

(57) Imino-substituierte Pyridine können durch Reduktion von entsprechenden Ketonen, und gegebenenfalls nachfolgender Verseifung, Cyclisierung, Hydrierung und Isomerisierung hergestellt werden. Die Imino-substituierten Pyridine können als Wirkstoffe in Arzneimitteln, insbesondere als HMG-CoA-Reduktaseinhibitoren eingesetzt werden.

EP 0 411 420 A2

# IMINO-SUBSTITUIERTE PYRIDINE

Die Erfindung betrifft imino-substituierte Pyridine, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl-Coenzym A Reduktase (HMG-CoA-Reduktase) sind [Mevinolin, EP-A 22 478; US 4 231 938]. Außerdem sind in der DOS 38.01.406 substituierte Pyridine beschrieben. Ferner sind 3-Desmethyl-mevalonsäurederivate bekannt (vgl. DE 3 823 045 A 1).

Es wurden nun imino-substituierte Pyridine der allgemeinen Formel (I)

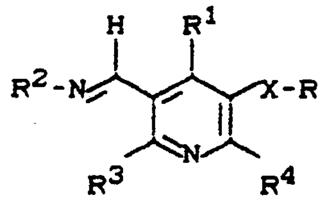

in welcher

$R^1$ für Aryl mit 6 bis 10 Kohlenstoffatomen steht, das gegebenenfalls bis zu 3-fach gleich oder verschieden durch Halogen, Hydroxy, Trifluormethyl, Trifluormethoxy, Nitro, Cyano, geradkettiges oder verzweigtes Alkyl oder Alkoxy mit jeweils bis zu 8 Kohlenstoffatomen oder durch Aryloxy mit 6 bis 10 Kohlenstoffatomen substituiert ist,

$R^2$ - für Aryl mit 6 bis 10 Kohlenstoffatomen steht, das gegebenenfalls bis zu 3-fach gleich oder verschieden durch geradkettiges oder verzweigtes Alkyl mit jeweils bis zu 8 Kohlenstoffatomen, Halogen, Cyano, Trifluormethyl, Trifluormethoxy oder Nitro substituiert ist, oder

- für geradkettiges oder verzweigtes Alkyl mit bis zu 10 Kohlenstoffatomen steht, das gegebenenfalls durch Aryl mit 6 bis 10 Kohlenstoffatomen, Hydroxy oder Alkoxy mit bis zu 8 Kohlenstoffatomen substituiert ist, oder

- für eine Gruppe der Formel $-OR^5$ steht, worin

$R^5$ - Wasserstoff oder geradkettiges oder verzweigtes Alkyl mit bis zu 8 Kohlenstoffatomen bedeutet, das gegebenenfalls durch Aryl mit 6 bis 10 Kohlenstoffatomen substituiert ist,

oder

- Aryl mit 6 bis 10 Kohlenstoffatomen bedeutet, das gegebenenfalls durch geradkettiges oder verzweigtes Alkyl oder Alkoxy mit jeweils bis zu 8 Kohlenstoffatomen, Halogen, Nitro, Cyano, Trifluormethyl oder Trifluormethoxy substituiert ist,

$R^3$ - für Cycloalkyl mit 3 bis 8 Kohlenstoffatomen steht, oder

- für Aryl mit 6 bis 10 Kohlenstoffatomen steht, das gegebenenfalls bis zu 3-fach gleich oder verschieden durch Halogen oder durch geradkettiges oder verzweigtes Alkyl mit bis zu 8 Kohlenstoffatomen substituiert ist, oder

- für geradkettiges oder verzweigtes Alkyl mit bis zu 10 Kohlenstoffatomen steht, das gegebenenfalls durch eine Gruppe der Formel $-OR^5$ substituiert ist,

worin

$R^5$ die oben angegebene Bedeutung hat,

$R^4$ - für geradkettiges oder verzweigtes Alkyl mit bis zu 10 Kohlenstoffatomen steht, oder

- für Cycloalkyl mit 3 bis 8 Kohlenstoffatomen steht

X - für eine Gruppe der Formel $-CH_2-CH_2-$ oder $-CH=CH-$steht,

und

R - für eine Gruppe der Formel

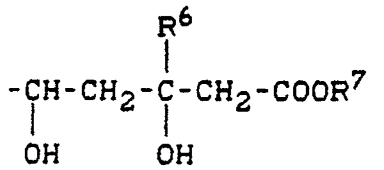 oder 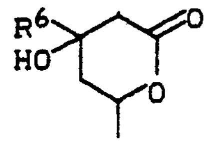 steht,

worin

$R^6$ - Wasserstoff oder geradkettiges oder verzweigtes Alkyl mit bis zu 10 Kohlenstoffatomen bedeutet und

$R^7$ - Wasserstoff oder geradkettiges oder verzweigtes Alkyl mit bis zu 10 Kohlenstoffatomen bedeutet, das durch Phenyl substituiert sein kann, oder

- Aryl mit 6 bis 10 Kohlenstoffatomen oder ein Kation bedeutet,

und deren Salze gefunden.

Bildet $R^7$ mit der Carboxygruppe einen Esterrest so ist hiermit bevorzugt ein physiologisch verträglicher Esterrest gemeint, der in vivo leicht zu einer freien Carboxylgruppe und einem entsprechenden physiologisch verträglichen Alkohol hydrolysiert wird. Hierzu gehören beispielsweise Alkylester ($C_1$ bis $C_6$) und Aralkylester ($C_7$ bis $C_{10}$), bevorzugt ($C_1$ bis $C_4$)-Alkylester sowie Benzylester. Darüberhinaus seien die folgenden Esterreste genannt: Methylester, Ethylester, Propylester, Benzylester.

Steht $R^7$ für ein Kation, so ist bevorzugt ein physiologisch verträgliches Metall- oder Ammoniumkation gemeint. Bevorzugt sind hierbei Alkali- bzw. Erdalkalikationen wie beispielsweise Natrium-, Kalium-, Magnesium- oder Calciumkationen, sowie Aluminium- oder Ammoniumkationen, sowie nichttoxische substituierte Ammoniumkationen aus Aminen wie ($C_1$-$C_4$)-Dialkylamine, ($C_1$-$C_4$)-Trialkylamine, Prokain, Dibenzylamin, N,N′-Dibenzylethylendiamin, N-Benzyl-ß-phenylethylamin, N-Methylmorpholin oder N-Ethylmorpholin, 1-Ephenamin, Dihydroabietylamin, N,N′-Bis-dihydroabietylethylendiamin, N-Niederalkylpiperidin und andere Amine, die zur Bildung von Salzen verwendet werden können.

Überraschenderweise zeigen die erfindungsgemäßen iminosubstituierten Pyridine eine überlegene inhibitorische Wirkung auf die HMG-CoA-Reduktase (3-Hydroxy-3-methylglutaryl-Coenzym A Reduktase).

Bevorzugt sind Verbindungen der allgemeinen Formel (I) in welcher

$R^1$ - für Phenyl steht, das gegebenenfalls bis zu 2-fach gleich oder verschieden durch Fluor, Chlor, Brom, Hydroxy, geradkettiges oder verzweigtes Alkyl oder Alkoxy mit jeweils bis zu 6 Kohlenstoffatomen oder durch Phenoxy substituiert ist,

$R^2$ - für Phenyl steht, das gegebenenfalls bis zu 2-fach gleich oder verschieden durch Fluor, Chlor, Brom oder durch geradkettiges oder verzweigtes Alkyl mit bis zu 6 Kohlenstoffatomen substituiert ist, oder - für geradkettiges oder verzweigtes Alkyl mit bis zu 8 Kohlenstoffatomen steht, das gegebenenfalls durch Phenyl oder Alkoxy mit bis zu 6 Kohlenstoffatomen substituiert ist, oder

- für eine Gruppe der Formel $-OR^5$ steht

worin

$R^5$ - Wasserstoff oder geradkettiges oder verzweigtes Alkyl mit bis zu 6 Kohlenstoffatomen bedeutet, das gegebenenfalls durch Phenyl substituiert ist,

oder

- Phenyl bedeutet, das gegebenenfalls durch geradkettiges oder verzweigtes Alkyl oder Alkoxy mit jeweils bis zu 6 Kohlenstoffatomen substituiert ist,

$R^3$ - für Cyclopropyl, Cyclobutyl, Cyclopentyl oder Cyclohexyl steht, oder

- für Phenyl steht, das gegebenfalls bis zu 2-fach gleich oder verschieden durch Fluor, Chlor, Brom oder durch geradkettiges oder verzweigtes Alkyl mit bis zu 6 Kohlenstoffatomen substituiert ist, oder

- für geradkettiges oder verzweigtes Alkyl mit bis zu 8 Kohlenstoffatomen steht, das gegebenenfalls durch eine Gruppe der Formel $-OR^5$ substituiert ist worin

$R^5$ die oben angegebene Bedeutung hat,

$R^4$ - für geradkettiges oder verzweigtes Alkyl mit bis zu 8 Kohlenstoffatomen steht, oder

- für Cyclopropyl, Cyclopentyl oder Cyclohexyl steht,

X - für eine Gruppe der Formel $-CH_2-CH_2-$ oder $-CH=CH-$steht

und

R - für eine Gruppe der Formel

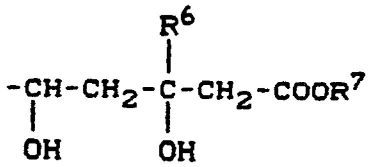 oder 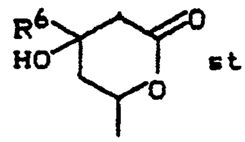 steht,

worin

$R^6$ - Wasserstoff oder geradkettiges oder verzweigtes Alkyl mit bis zu 8 Kohlenstoffatomen bedeutet und

$R^7$ - Wasserstoff oder geradkettiges oder verzweigtes Alkyl mit bis zu 8 Kohlenstoffatomen oder Benzyl bedeutet, oder

- Phenyl oder ein Kation bedeutet, und deren Salze.

Besonders bevorzugt sind Verbindungen der allgemeinen Formel (I), in welcher

$R^1$ - für Phenyl steht, das gegebenenfalls durch Fluor, Chlor, geradkettiges oder verzweigtes Alkyl mit bis zu 4 Kohlenstoffatomen oder Phenoxy substituiert ist, oder

$R^2$ - für eine Gruppe der Formel $-OR^5$ steht worin

$R^5$ - Wasserstoff, Benzyl oder geradkettiges oder verzweigtes Alkyl mit bis zu 4 Kohlenstoffatomen bedeutet oder

- Phenyl bedeutet,

$R^3$ - für Cyclopropyl oder Phenyl steht, oder

- für geradkettiges oder verzweigtes Alkyl mit bis zu 6 Kohlenstoffatomen steht, das gegebenenfalls durch eine Gruppe der Formel $-OR^5$ substituiert ist worin

$R^5$ - die oben angegebene Bedeutung hat,

$R^4$ - für geradkettiges oder verzweigtes Alkyl mit bis zu 6 Kohlenstoffatomen oder Cyclopropyl steht,

X - für eine Gruppe -CH=CH- steht

und

R - für eine Gruppe der Formel

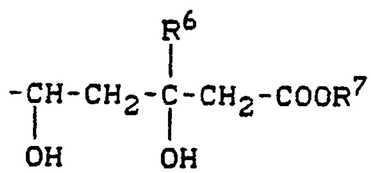 oder 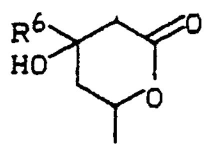 steht,

worin

$R^6$ - Wasserstoff, Methyl, Ethyl, Propyl, Isopropyl, Butyl, Isobutyl oder tert.Butyl bedeutet und

$R^7$ - Wasserstoff, Methyl, Ethyl, Propyl, Isopropyl, Butyl, Isobutyl, tert.Butyl oder Benzyl bedeutet, oder

- ein Natrium-, Kalium-, Calcium-, Magnesium-oder Ammoniumion bedeutet und deren Salze.

Insbesondere bevorzugt steht $R^6$ für Wasserstoff.

Die erfindungsgemäßen imino-substituierten Pyridine der allgemeinen Formel (I) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der Gruppe X bzw. des Restes R ergeben sich unterschiedliche Stereoisomere, die im folgenden näher erläutert werden sollten:

a) Steht die Gruppe -X- für eine Gruppe der Formel -CH=CH- , so können die erfindungsgemäßen Verbindungen in zwei stereoisomeren Formen existieren, die an der Doppelbindung E-konfiguriert (II) oder Z-konfiguriert (III) sein können:

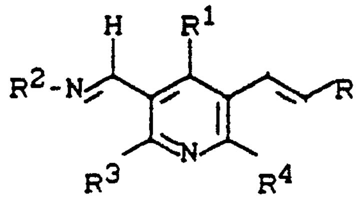

(II) E-Form

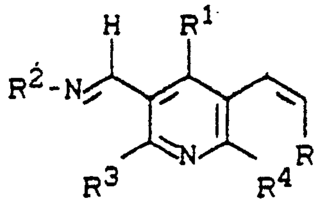

(III) Z-Form

($R^1$, $R^2$, $R^3$ und $R^4$ haben die oben angegebene Bedeutung).

Bevorzugt sind diejenigen Verbindungen der allgemeinen Formel (I) die E-konfiguriert sind (II).

b) Steht der Rest -R- für eine Gruppe der Formel

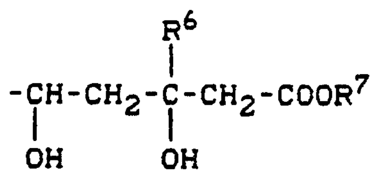

so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

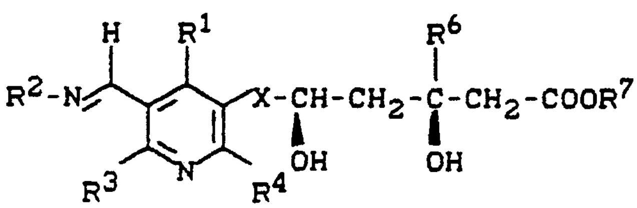

Erythro-Form (IV)

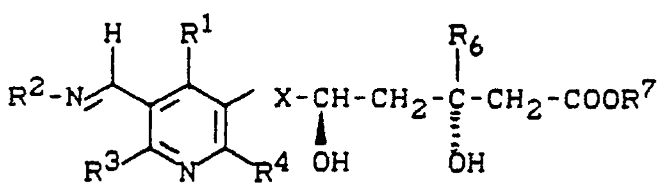

Threo-Form (V)

Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).

Bevorzugt sind hierbei die Erythro-konfigurierten Isomeren, besonders bevorzugt das 3R,5S-Isomere sowie das 3R,5S-3S,5R-Racemat.

c) Steht der Rest -R- für eine Gruppe der Formel

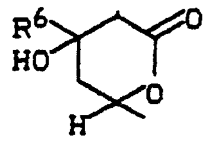

so besitzen die imino-substituierten Pyridine mindestens zwei asymmetrische Kohlenstoffatome, nämlich das Kohlenstoffatom an dem die Hydroxygruppe gebunden ist, und das Kohlenstoffatom an welchem der Rest der Formel

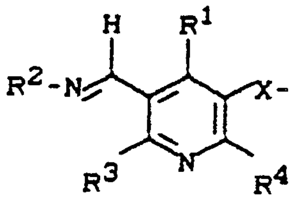

gebunden ist. Je nach der Stellung der Hydroxygruppe zur freien Valenz am Lactonring können die imino-substituierten Pyridine als cis-Lactone (VI) oder als trans-Lactone (VII) vorliegen.

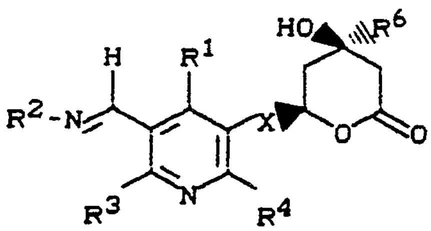

cis-Lacton (VI)

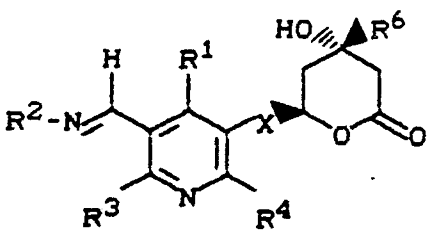

trans-Lacton (VII)

Sowohl vom cis-Lacton aus als auch vom trans-Lacton existieren wiederum jeweils zwei Isomere nämlich das 4R,6R-Isomere bzw. das 4S,6S-Isomere (cis-Lacton),
sowie das 4R,6S-Isomere bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat. Beispielsweise seien die folgenden isomeren Formen der imino-substituierten Pyridine genannt:

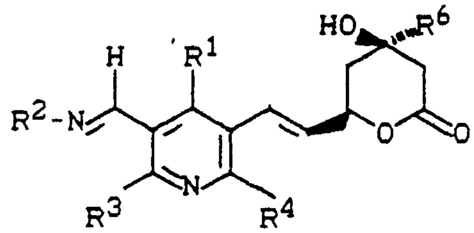

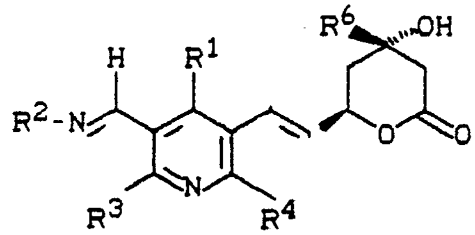

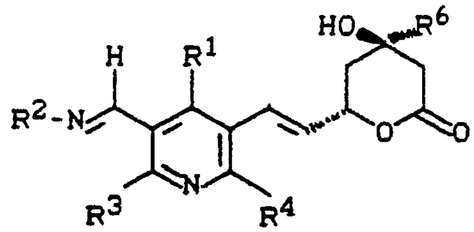

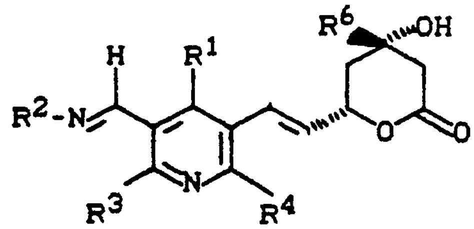

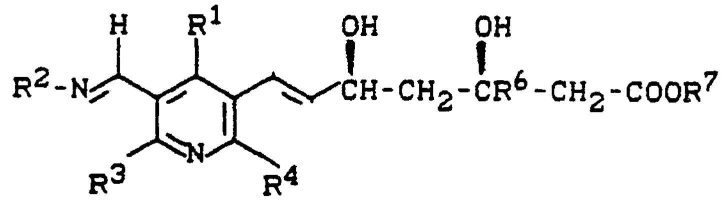

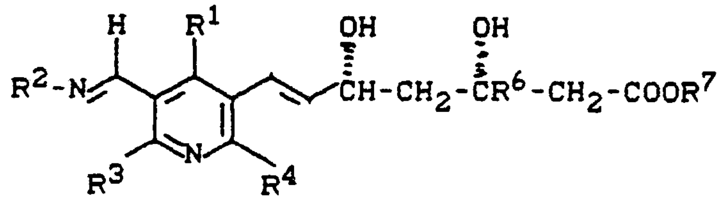

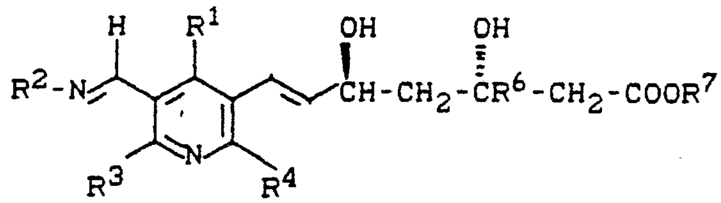

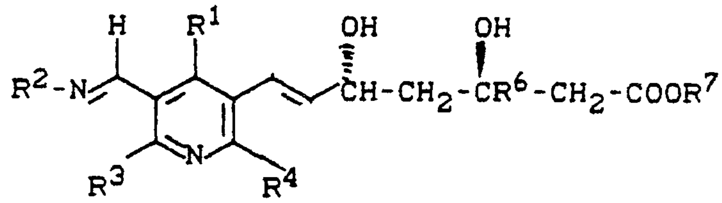

Außerdem wurde ein Verfahren zur Herstellung der iminosubstituierten Pyridine der allgemeinen Formel (I)

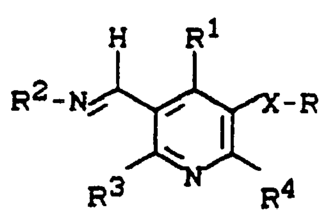

(I)

in welcher
$R^1$, $R^2$, $R^3$, $R^4$, X und R die oben angegebene Bedeutung haben,
gefunden, das dadurch gekennzeichnet ist, daß man Ketone der allgemeinen Formel (VIII)

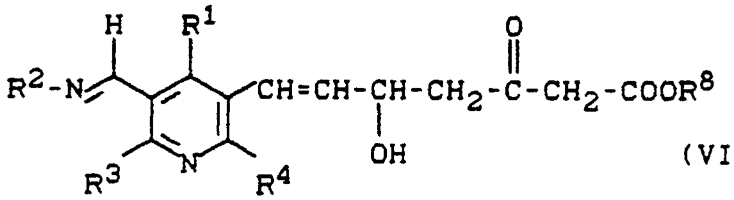

(VIII)

,

in welcher
$R^1$, $R^2$, $R^3$, $R^4$, X und R die oben angegebene Bedeutung haben
und
$R^8$ - für Alkyl steht,
reduziert,
im Fall der Herstellung der Säuren die Ester verseift, im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (x = -CH=CH-) nach üblichen Methoden hydriert,
und gegebenenfalls Isomeren trennt.

Das erfindungsgemäße Verfahren kann durch das folgende Formelschema erläutert werden:

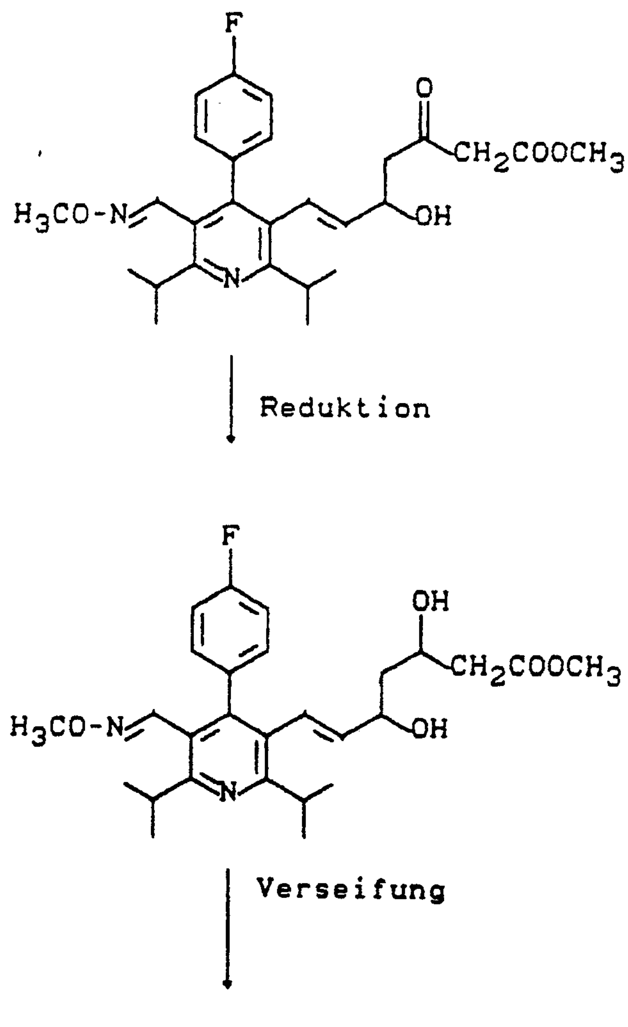

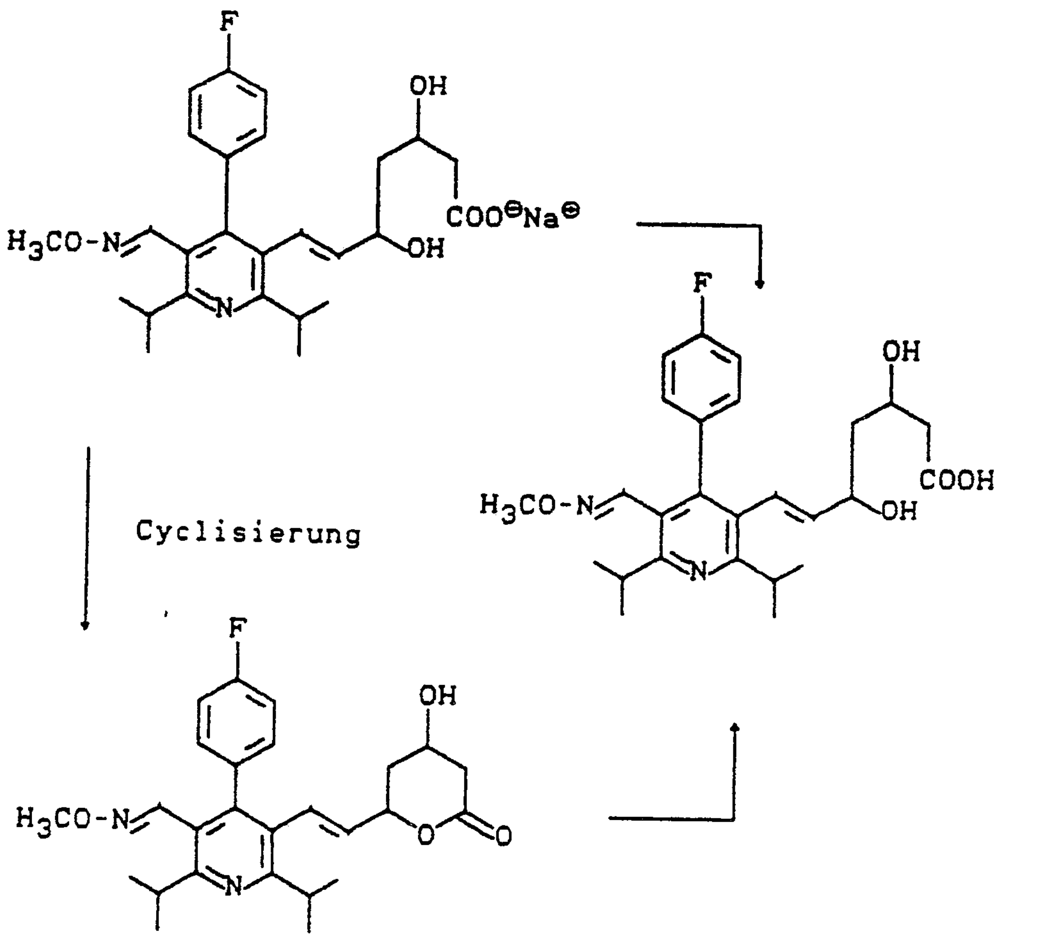


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-boranaten, Natrium-trialkyl-hydrido-boranaten, Natrium-cyano-trihydrido-boranat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispiels weise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80° C bis +3° C, bevorzugt von -78° C bis 0° C.

Die Reduktion wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (VIII) unter solchen Bedingungen durchgeführt werden, unter denen sowohl die Carbonylgruppe als auch die Doppelbindung reduziert wird.

Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ic)

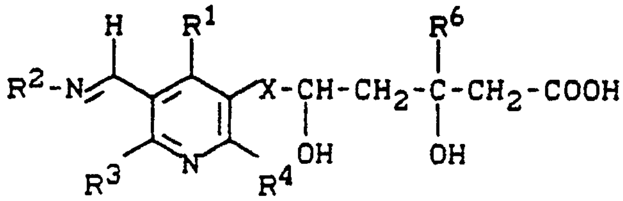 (Ic)

in welcher
$R^1$, $R^2$, $R^3$, $R^4$, $R^6$ und X die oben angegebene Bedeutung haben.

Die Carbonsäureester im Rahmen der allgemeinen Formel (I) entsprechen der Formel (Id)

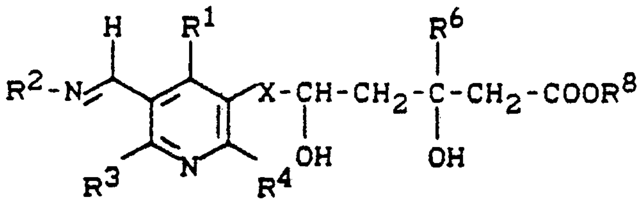 (Id)

in welcher
$R^1$, $R^2$, $R^3$, $R^4$, $R^6$, $R^8$ und X die oben angegebene Bedeutung haben.

Die Salze der erfindungsgemäßen Verbindungen im Rahmen der allgemeinen Formel (I) entsprechen der Formel (Ie)

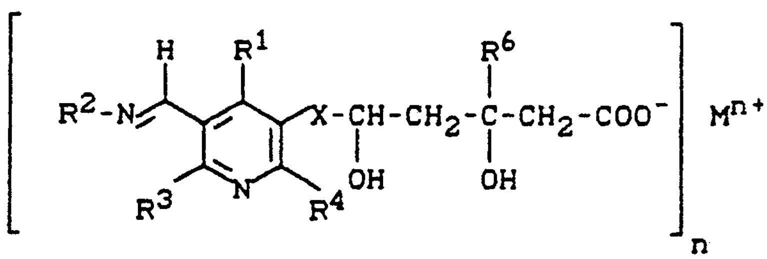 (Ie)

in welcher
$R^1$, $R^2$, $R^3$, $R^4$, $R^6$ und X die oben angegebene Bedeutung haben,
und
$M^{n+}$ für ein Kation steht, wobei n die Wertigkeit angibt.

Die Lactone im Rahmen der allgemeinen Formel (I) entsprechen der Formel (If)

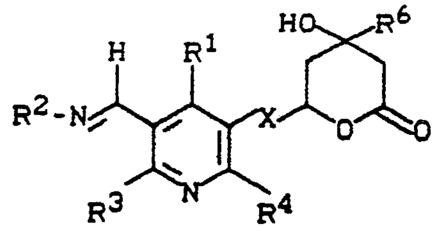

(If)

in welcher
$R^1$, $R^2$, $R^3$, $R^4$, $R^6$ und X die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar)

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Verseifung entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säu ren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäuren. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel zur Cyclisierung eignen sich Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40 °C bis +200 °C, bevorzugt von -25 °C bis +50 °C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N′-Dicyclohexylcarbodiimid-paratoluolsulfonat, N-Cyclohexyl-N′-[2-(N″-methylmorpholinium)ethyl]carbodiimid oder H-(3-Dimethylaminopropyl)-N′-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Ganz besonders bevorzugt werden Chlorkohlenwasserstoffe wie beispielsweise Methylenchlorid, Chloroform oder Tetrachlorkohlenstoff eingesetzt.

Die Cyclisierung wird im allgemeinen in einem Temperaturbereich von 0°C bis +80C° bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Ester. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ig)

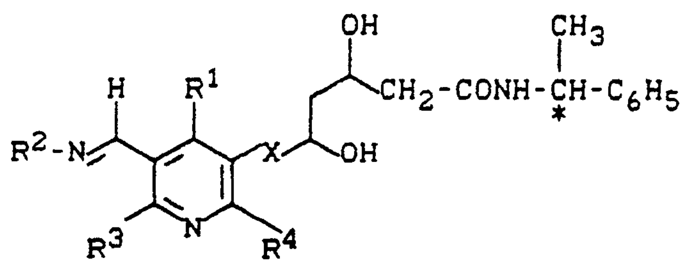

(Ig)

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kalium hydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden:

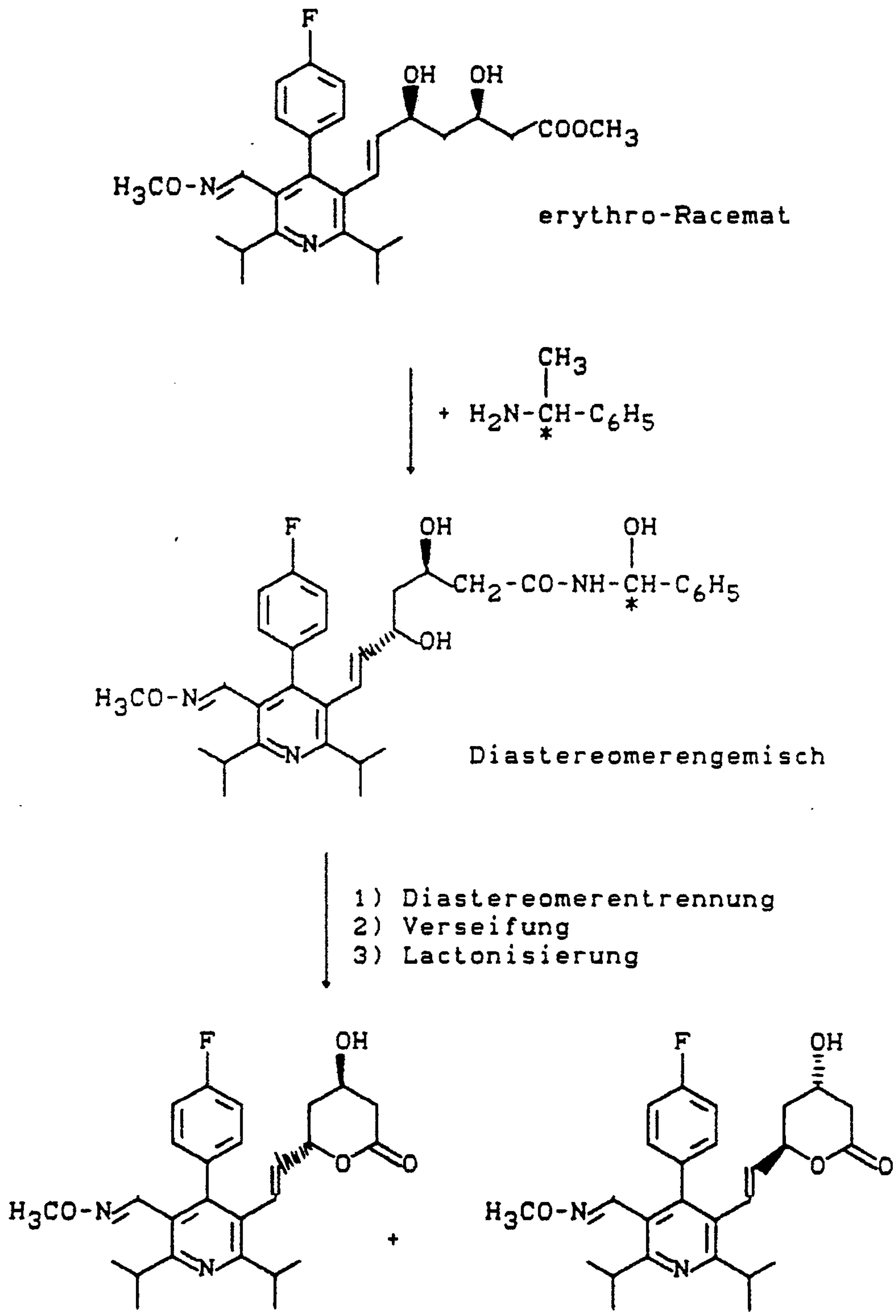


Die als Ausgangsstoffe eingesetzten Ketone (VIII) sind neu.

Es wurde ein Verfahren zur Herstellung der erfindungsgemäßen Ketone der allgemeinen Formel (VIII)

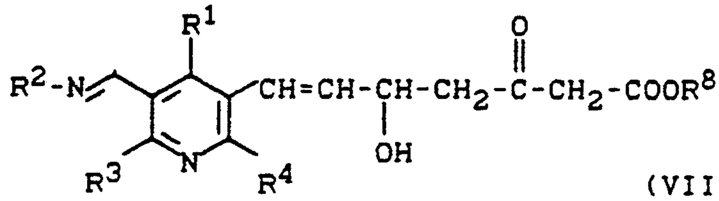

(VIII)

in welcher
$R^1$, $R^2$, $R^3$ und $R^8$ die oben angegebene Bedeutung haben, gefunden, das dadurch gekennzeichnet ist, daß man Aldehyde der allgemeinen Formel (IX)

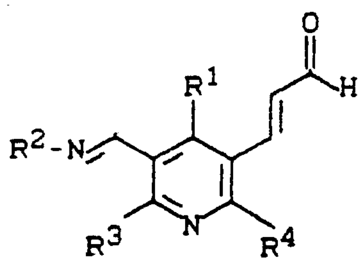

(IX)

in welcher
$R^1$, $R^2$, $R^3$, und $R^4$ die oben angegebene Bedeutung haben, in inerten Lösemitteln mit Acetessigester der allgemeinen Formel (X)

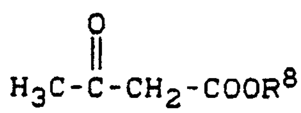

(X)

in welcher
$R^8$ die oben angegebene Bedeutung hat,
in Anwesenheit von Basen umsetzt.

Das erfindungsgemäße Verfahren kann beispielsweise durch folgendes Formelschema erläutert werden:

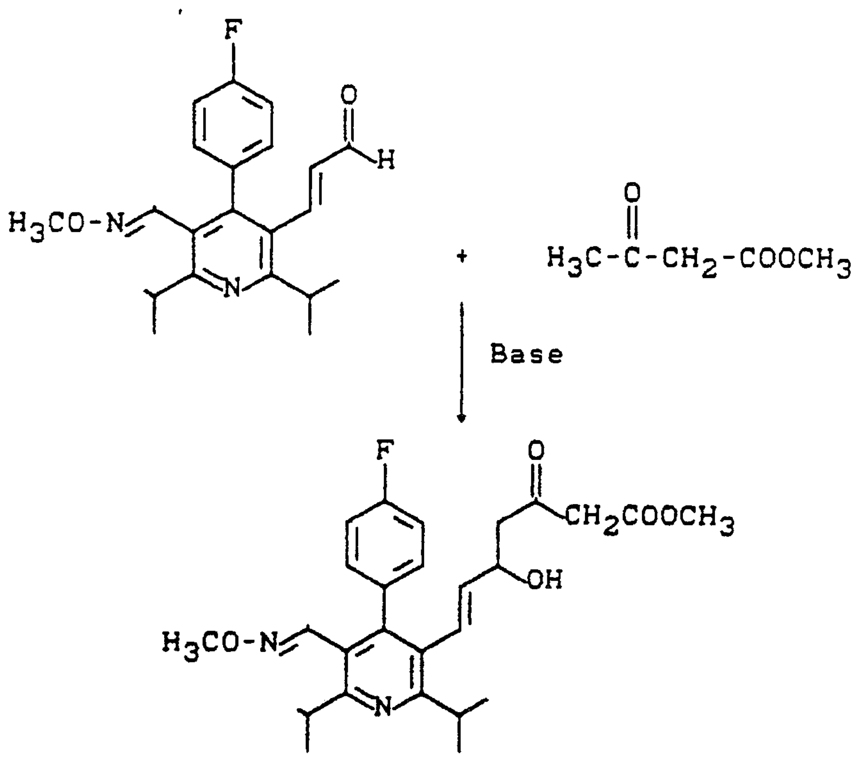


Als Basen kommen hierbei die üblichen stark basischen Verbindungen in Frage. Hierzu gehören bevorzugt lithiumorganische Verbindungen wie beispielsweise n-Butyllithi um, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden n-Butyllithium oder Natriumhydrid oder deren Gemisch eingesetzt.

Eventuell sind Zusätze von Metallhalogeniden wie z.B. Magnesiumchlorid, Zinkchlorid oder Zinkbromid vorteilhaft. Besonders bevorzugt ist der Zusatz von Zinkhalogeniden.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erdölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80 °C bis +50 °C, bevorzugt von -20 °C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:
Acetessigsäuremethylester, Acetessigsäureethylester,
Acetessigsäurepropylester, Acetessigsäureisopropylester.

Die Herstellung der als Ausgangsstoffe eingesetzten Aldehyde der allgemeinen Formel (IX) soll im folgenden Schema [A] beispielhaft erläutert werden.

[A]

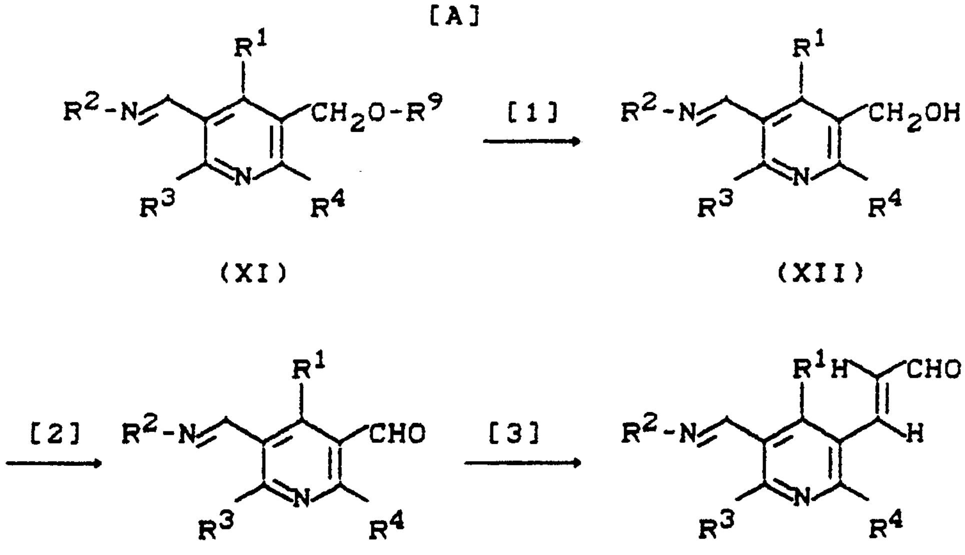


(XI) (XII)

(XIII) (IX)

Hierbei werden die imino-substituierten Pyridine der Formel (XI),in welchen $R^9$ für eine typische Hydroxyschutzgruppe, beispielsweise für den tert.-Butyldimethylsilyl-Rest steht, im ersten Schritt [1] in inerten Lösungsmitteln wie Ethern, beispielsweise Diethylether, Tetrahydrofuran oder Dioxan, vorzugsweise Tetrahydrofuran, unter Abspaltung des Restes $R^9$ nach üblicher Methode, beispielsweise mit Tetrabutylammoniumfluorid, in die Hydroxymethylverbindungen (XII) überführt. Die Reaktion verläuft in einem Temperaturbereich von -10°C bis +60°C, vorzugsweise bei 0°C bis +30°C.

Die Hydroxymethylverbindungen (XII) werden im zweiten Schritt [2] nach üblichen Methoden zu den Aldehyden (XIII) oxidiert. Die Oxidation kann beispielsweise mit Pyridiniumchlorochromat, gegebenenfalls in Anwesenheit von Aluminiumoxid, in inerten Lösemitteln wie Chlorkohlenwasserstoffen, vorzugsweise Methylenchlorid, in einem Temperaturbereich von 0°C bis 60°C, bevorzugt bei Raumtemperatur durchgeführt werden, oder aber mit Trifluoressigsäure/Dimethylsulfoxid nach den üblichen Methoden der Swern Oxidation durchgeführt werden. Die Aldehyde (XIII) werden im dritten Schritt [3] mit Diethyl-2-(cyclohexylamino)-vinylphosphonat in Anwesenheit von Natriumhydrid in inerten Lösemitteln wie

Ethern, beispielsweise Diethylether, Tetrahydrofuran oder Dioxan, vorzugsweise in Tetrahydrofuran, in einem Temperaturbereich von -20°C bis +40°C, vorzugsweise von -5°C bis Raumtemperatur zu den Aldehyden (IX) umgesetzt.

Die als Ausgangsstoffe eingesetzten substituierten Pyridine der Formel (XI) sind neu. Man erhält sie im allgemeinen gemäß Schema [B],

indem man

[B] Verbindungen der allgemeinen Formel (XIV)

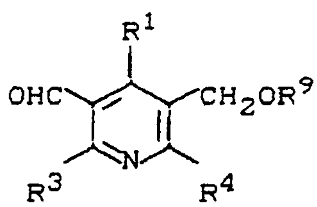

(XIV),

in welcher

$R^1$, $R^3$, $R^4$ und $R^9$ die oben angegebene Bedeutung haben

mit Aminen oder Hydroxylaminderivaten der allgemeinen Formel (XV)

$H_2N\text{-}R^2$ (XV)

in welcher

$R^2$ die oben angegebene Bedeutung hat

in einem der oben aufgeführten Lösungsmitteln, vorzugsweise Methylenchlorid, in einem Temperaturbereich 0° C bis +70° C, vorzugsweise bei Raumtemperatur umsetzt.

Die Verbindungen der allgemeinen Formel (XIV) sind an sich bekannt oder können nach bekannter Methode hergestellt werden (vgl. DOS 3 801 406).

Die Verbindungen der allgemeinen Formel (XV) sind ebenfalls bekannt (vgl. Beilstein 1, 288, Houben-Weyl's "Methoden der organischen Chemie", Bd.- XII 1 und XII 2).

Die erfindungsgemäßen Verbindungen der allgemeinen Formel (I) besitzen wertvolle pharmakologische Eigenschaften und können in Arzneimitteln eingesetzt werden. Insbesondere sind sie Inhibitoren der 3-Hydroxy-3-methylglutaryl-Coenzym A (HMG-CoA) Reduktase und infolge dessen Inhibitoren der Cholesterolbiosynthese. Sie können deshalb zur Behandlung von Hyperlipoproteinämie, Lipoproteinämie oder Atherosklerose eingesetzt werden. Die erfindungsgemäßen Wirkstoffe bewirken außerdem eine Senkung des Cholesteringehaltes im Blut.

Die Enzymaktivitätsbestimmung wurde modifiziert nach G. C. Ness et al., Archives of Biochemistry and Biophysics 197 , 493 - 499 (1979) durchgeführt. Männliche Ricoratten (Körpergewicht 300 - 400 g) wurden 11 Tage mit Altrominpulverfutter, dem 40 g Colestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat, 0,03 m Ethylendiamintetraessigsäure, 0,002 m Dithiothreit (SPE)-Puffer pH 7,2, homogenisiert. Anschließend wurde 15 Minuten bei 15.000 g zentrifugiert und das Sediment verworfen. Der Überstand wurde 75 Minuten bei 100.000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumens SPE-Puffer aufgenommen, homogenisiert und bei -78° C eingefroren und gelagert (= Enzym-lösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37° C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase 35 µMol $K_xH_y$-Phosphat pH 7,2, 20 µl Enzympräparation und 56 nMol 3-Hydroxy-3-methyl-glutaryl Coenzym A (Glutaryl-3-$^{14}C$) 100.000 dpm.

Nach Inkubation von 60 Minuten bei 37° C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. $IC_{50}$-Werte wurden durch Auftrag der prozentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 1 gesetzt und mit dem simultan bestimmten $IC_{50}$-Wert der Testverbindung verglichen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin

können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchem die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Ausgangsverbindungen

### Beispiel I

(E/Z)-4-Carboxyethyl-5-(4-fluorphenyl )-2-methyl-pent-4-en-3-on

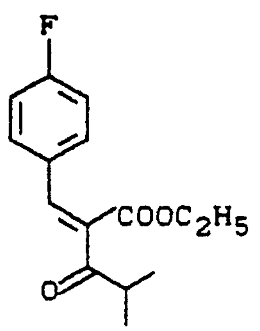

62 g (0,5 Mol) 4-Fluorbenzaldehyd und 79 g (0,5 mol) Isobutyrylessigsäureethylester werden in 300 ml Isopropanol vorgelegt und mit einem Gemisch aus 2,81 ml (28 mmol) Piperidin und 1,66 ml (29 mmol) Essigsäure in 40 ml Isopropanol versetzt. Man läßt 48 Stunden bei Raumtemperatur rühren, engt im Vakuum ein und destilliert den Rückstand im Hochvakuum.

Kp 0,5 mm: 127 °C

Ausbeute: 108,7 g (82,3% der Theorie)

### Beispiel II

1,4-Dihydro-2,6-diisopropyl-4-(4-fluorphenyl)-pyridin-3,5-dicarbonsäure-diethylester

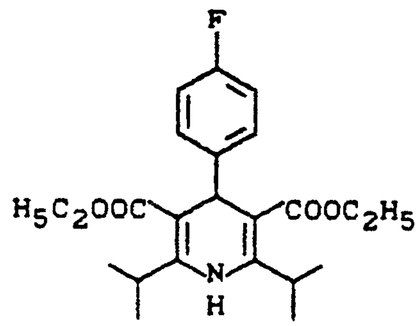

98 g (0,371 mol) der Verbindung aus Beispiel I werden mit 58,3 g (0,371 mol) 3-Amino-4-methyl-pent-2-en-säure-ethylester in 300 ml Ethanol 18 h am Rückfluß gekocht. Die Mischung wird auf Raumtemperatur abgekühlt, das Lösungsmittel im Vakuum abgedampft und die nicht umgesetzten Ausgangsmaterialien im Hochvakuum bei 130 °C abdestilliert. Den zurückbleibenden Sirup verrührt man mit n-Hexan und saugt den ausgefallenen Niederschlag ab, wäscht mit n-Hexan nach und trocknet im Exsikkator,
Ausbeute: 35 g (23,4% der,Theorie)
$^1$H-NMR ($CDCl_3$): δ 1,1 - 1,3 (m, 18H); 4,05 - 4,25 (m, 6H); 5,0 (s, 1H); 6,13 (s, 1H); 6,88 (m, 2H); 7,2 (m, 2H) ppm.

<u>Beispiel III</u>

2,6-Diisopropyl-4-(4-fluorphenyl)-pyridin-3,5-dicarbonsäure-diethylester

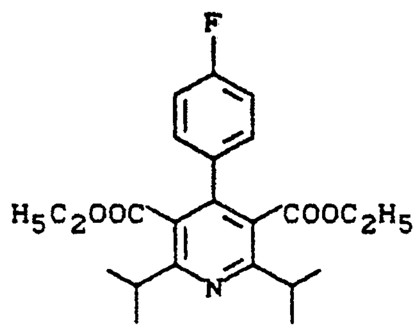

Zu einer Lösung von 6,6 g (16,4 mmol) der Verbindung aus Beispiel II in 200 ml Methylenchlorid p.A. gibt man 3,8 g (16,4 mmol) 2,3-Dichlor-5,6-dicyan-p-benzochinon und rührt 1 h bei Raumtemperatur. Dann wird über Kieselgur abgesaugt, die Methylenchloridphase dreimal mit je 100 ml Wasser extrahiert und über Magnesiumsulfat getrocknet. Nach Einengen im Vakuum wird der Rückstand an einer Säule (100 g Kieselgel 70-230 mesh,

ø 3,5 cm,

mit Essigester/Petrolether 1:9) chromatographiert.
Ausbeute: 5,8 g (87,9% der Theorie)
$^1$H-NMR ($CDCl_3$): = δ 0, 98 (t, 6H); 1,41 (d, 12H); 3,1 (m, 2H); 4,11 (q, 4H); 7,04 (m, 2H); 7,25 (m, 2H) ppm.

<u>Beispiel IV</u>

2,6-Diisopropyl-4-(4-fluorphenyl)-5-hydroxymethyl-pyridin-3-carbonsäure-ethylester

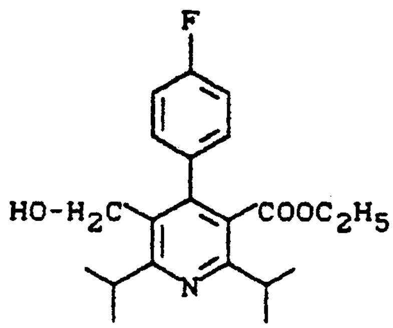

Unter Stickstoff gibt man zu einer Lösung von 9,2 g (23 mmol) der Verbindung aus Beispiel III in 100 ml trockenem Tetrahydrofuran bei -10 °C bis -50 °C 21 ml (80,5 mmol) einer 3,5 molaren Lösung von Natrium-bis-(2-methoxyethoxy)-dihydroaluminat in Toluol und rührt 5 h bei Raumtemperatur. Nach Abkühlen auf 0 °C tropft man vorsichtig 100 ml Wasser zu und extrahiert dreimal mit je 100 ml Essigester. Die vereinigten organischen Phasen werden mit gesättigter Natriumchlorid-Lösung gewaschen, über Magnesiumsulfat getrocknet und im Vakuum eingedampft. Der Rückstand wird an einer Säule (200 g Kieselgel 70-230 mesh,

ø 4,5 cm,

mit Essigester/Petrolether 3:7) chromatographiert.
Ausbeute: 7,2 g (87,2% der Theorie)
$^1H$-NMR ($CDCl_3$): δ = 0,95 (t, 3H); 1,31 (m, 12H); 3,05 (m, 1H); 3,48 (m, 1H), 3,95 (q, 2H); 4,93 (d, 2H); 7,05 - 7,31 (m, 4H) ppm.

Beispiel V

5-(tert.Butyldimethylsilyloxymethyl)-2,6-diisopropyl-4-(4-fluorphenyl)-pyridin-3-carbonsäure-ethylester

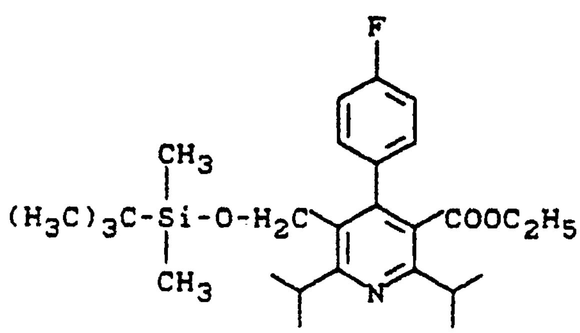

Zu einer Lösung von 4,5 g (12,5 mmol) der Verbindung aus Beispiel IV in 50 ml Dimethylformamid gibt man bei Raumtemperatur 2,1 g (13,8 mmol) tert.Butyldimethylsilylchlorid, 1,8 g (27,5 mmol) Imidazol und 0,05 g 4-Dimethylaminopyridin. Es wird über Nacht bei Raumtemperatur gerührt, mit 200 ml Wasser versetzt und mit 1 N Salzsäure auf pH 3 eingestellt. Die Mischung wird dreimal mit je 100 ml Ether extrahiert, die vereinigten organischen Phasen einmal mit gesättigter Natriumchlorid-Lösung gewaschen, über Magnesiumsulfat getrocknet und im Vakuum eingeengt. Der Rückstand wird an einer Säule (150 g Kieselgel, 70-230 mesh,

ø 4 cm,

mit Essigester/Petrolether 1:9) chromatographiert.
Ausbeute: 4,2 g (73,7% der Theorie)
$^1H$-HMR ($CDCl_3$): δ 0,0 (s, 6H); 0,9 1 (s, 9H); 1,02 (t, 3H); 1,35 (m, 12H); 3,1 (m, 1H); 3,47 (m, 1H); 4,03 (q, 2H); 4,4 (s, 2H); 7,05 - 7,40 (m, 4H) ppm.

Beispiel VI

3-(tert.Butyldimethylsilyloxymethyl)-2,6-diisopropyl-4-(4-fluorphenyl)-5-hydroxymethyl-pyridin

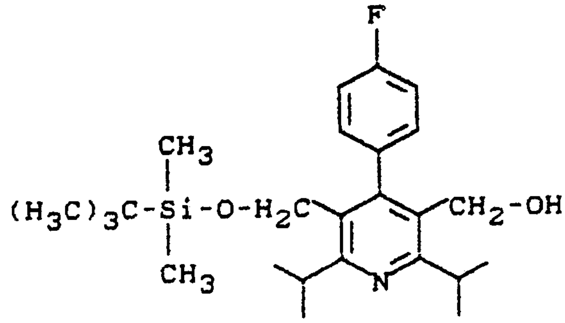

Unter Stickstoff gibt man zu einer Lösung von 4,2 g (9,2 mmol) der Verbindung aus Beispiel V in 100 ml trockenem Tetrahydrofuran bei 0°C 9,2 ml (32,2 mmol) einer 3,5 molaren Lösung von Natrium-bis-(2-methoxyethoxy)-dihydro-aluminat in Toluol und rührt über Nacht bei Raumtemperatur. Nach Abkühlen auf 0°C tropft man vorsichtig 100 ml Wasser zu und extrahiert dreimal mit je 100 ml Essigester. Die vereinigten organischen Phasen werden einmal mit gesättigter Natriumchlorid-Lösung gewaschen, über Magnesiumsulfat getrocknet und im Vakuum eingeengt. Der Rückstand wird an einer Säule (100 g Kieselgel 70-230 mesh,

ø 3,5 cm,

mit Essigester/Petrolether 2:8) chromatographiert.
Ausbeute: 2,4 g (60% der Theorie)
$^1$H-NMR ($CDCl_3$): $\delta$ = 0,2 (s, 6H); 1,11 (s, 9H); 1,6 (m, 12H); 3,7 (m, 2H); 4,55 (s, 2H); 4,65 (d, 2H); 7,35 - 7,55 (m, 4H) ppm.

Beispiel VII

5-(tert.Butyldimethylsilyloxymethyl )-2,6-diisopropyl-4-(4-fluorphenyl)-pyridin-3-carbaldehyd

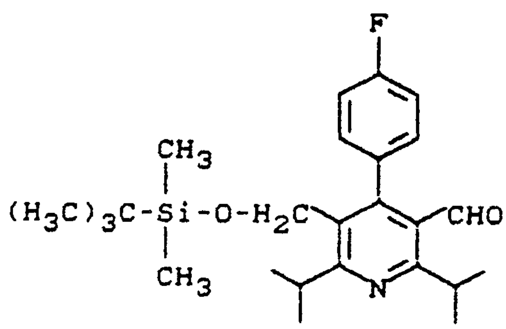

Zu einer Lösung von 2,7 g (6,2 mmol) der Verbindung aus Beispiel VI in 50 ml Methylenchlorid gibt man 1,24 g (12,4 mmol) neutrales Aluminiumoxid und 2,7 g (12,4 mmol) Pyridiniumchlorochromat und rührt 1 h bei Raumtemperatur. Man saugt über Kieselgur ab und wäscht mit 200 ml Methylenchlorid nach. Die Methylenchloridphase wird im Vakuum eingeengt und der Rückstand an einer Säule (100 g Kieselgel 70-230 mesh,

ø 3,5 cm,

mit Essigester/Petrolether 1:9) chromatographiert.
Ausbeute: 2 g (77% der Theorie) $^1$H-NMR ($CDCl_3$): $\delta$ = 0,0 (s, 6H); 0,9 (s, 9H); 1,35 (m, 12H); 3,5 (m, 1H); 3,9 (m, 1H); 4,38 (s, 2H); 7,15 - 7,35 (m, 4H); 9,8 (s, 1H) ppm.

Herstellungsbeispiele

Beispiel 1

3-(tert.-Butyldimethylsilyloxymethyl)-2,6-diisopropyl-4-(4-fluorphenyl)5-methoxyiminomethyl-pyridin

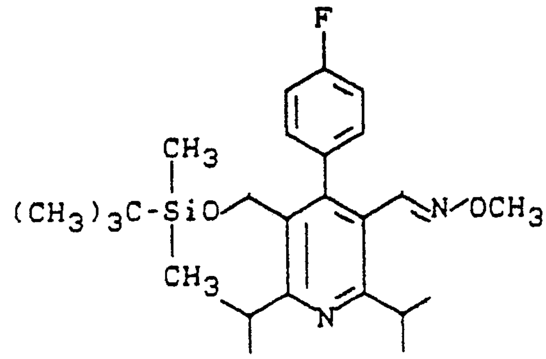

Zu einer Lösung von 2,1 g (5 mmol) der Verbindung aus Beispiel VII in 50 ml Ethanol p.A. gibt man 626 mg (7,5 mmol) O-Methylhydroxylamin Hydrochlorid und 0,6 ml (7,5 mmol) Pyridin und erhitzt 1 h unter Rückfluß. Nach dem Abkühlen auf Raumtemperatur wird am Rotationsverdampfer zur Hälfte eingeengt. Die beim weiteren Abkühlen auf 0° C ausfallenden Kristalle werden abgesaugt und getrocknet.
Ausbeute: 1,52 g (66,4 % der Theorie)
$^1$H-NMR ($CDCl_3$): $\delta$ = 0,01 (s, 6H); 0,91 (s, 9H); 1,48 (m, 2H); 3,50 (sept. 1H); 3,68 (sept. 1H); 3,89 (s, 3H); 4,39 (s, 2H); 7,10-7,30 (m, 4H); 7,82 (s, 1H) ppm.

Beispiel 2

2,6-Diisopropyl-4-(4-fluorphenyl)-3-hydroxymethyl-5-methoxyiminomethyl-pyridin

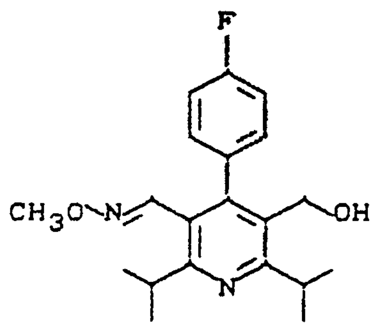

Zu einer Lösung von 1,5 g (3,3 mmol) der Verbindung aus Beispiel 1 in 15 ml absol. Tetrahydrofuran gibt man 3,3 ml (3,3 mmol) 1 M Tetrabutylammoniumfluorid-Lösung in Tetrahydrofuran und rührt 1 h bei Raumtemperatur. Die Reaktionslösung wird dann mit gesättigter Natriumhydrogencarbonat-Lösung versetzt und mehrmals mit Methylenchlorid extrahiert. Die vereinigten organischen Phasen werden getrocknet ($MgSO_4$), eingeengt und dann über Kieselgel filtriert.
Ausbeute: 1,07 g Rohprodukt (94,3% der Theorie)
$^1$H-NMR ($CDCl_3$): $\delta$ = 1,22 (d, 3H); 1,27 (d, 3H); 3,38 (sept., 1H); 3,48 (sept., 1H); 3,72 (s, 3H); 4,33 (d, 2H) 7,0-7,2 (m, 4H); 7,65 (s, 1H) ppm.

Beispiel 3

2,6-Diisopropyl-4-(4-fluorphenyl)-5-methoxyiminomethylpyridin-3-carbaldehyd

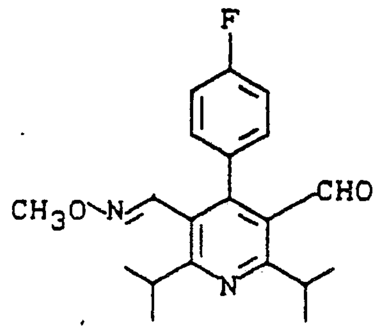

Zu einer Lösung von 1,05 g (3,05 mmol) der Verbindung aus Beispiel 2 in 50 ml Methylenchlorid gibt man 0,62 g (6,2 mmol) neutrales Aluminiumoxid und 1,3 g (6,1 mmol) Pyridiniumchlorochromat und rührt 1 h bei Raumtemperatur. Man saugt über Kieselgur ab und wäscht mit 200 ml Methylenchlorid nach. Die Methylenchloridphase wird im Vakuum eingeengt und der Rückstand an einer Säule (100 g Kieselgel, 70-230 mesh, Durchmesser 3,5 cm) mit Essigester/Petrolether 1:9 chromatographiert.
Ausbeute: 830 mg (79,6 % der Theorie)
$^1$H-MMR ($CDCl_3$): $\delta$ = 1,32 (d, 6H); 3,63 (sept., 1H); 3,84 (sept., 1H); 3,86 (s, 3H); 7,1-7,3 (m, 4H); 7,78 (s, 1H), 9,79 (s, 1H) ppm.

Beispiel 4

(E)-3-[2,6Diisopropyl-4-(4-fluorphenyl)-5-methoxyimino-methyl-pyrid-3-yl]-prop-2-enal

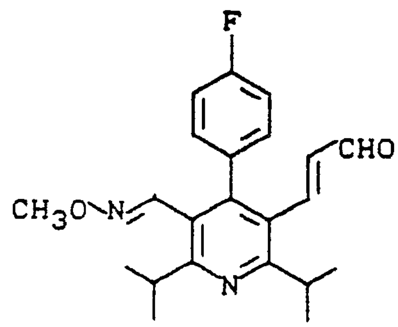

Unter Stickstoff tropft man zu einer Suspension von 110 mg (3,6 mmol) 80 %igem Natriumhydrid in 15 ml trockenem Tetrahydrofuran bei -5° C 750 mg (2,9 mmol) Diethyl-2-(cyclohexylamino)-vinylphosphonat gelöst in 30 ml trockenem Tetrahydrofuran. Nach 30 min werden bei derselben Temperatur 810 mg (2,4 mmol) der Verbindung aus Beispiel 3 in 40 ml trockenem Tetrahydrofuran zugetropft und 30 min zum Rückfluß erwärmt. Nach Abkühlen auf Raumtemperatur wird der Ansatz in 200 ml eiskaltes Wasser gegeben und dreimal mit je 100 ml Essigester extrahiert. Die vereinigten organischen Phasen werden mit gesättigter Natriumchlorid-Lösung gewaschen und über Magnesiumsulfat getrocknet. Nach Einengen im Vakuum wird der Rückstand in 70 ml Toluol aufgenommen, mit einer Lösung von 4,5 g (3,5 mmol) Oxalsäure-Dihydrat in 30 ml Wasser versetzt und 30 min zum Rückfluß erhitzt.

Nach Abkühlen auf Raumtemperatur werden die Phasen getrennt, die organische Phase mit gesättigter Natriumchlorid-Lösung gewaschen, über Magnesiumsulfat getrocknet und im Vakuum eingeengt. Der Rückstand wird an einer Säule (100 g Kieselgel, 70-230 mesh, Durchmesser 3,5 cm) mit Essigester/Petrolether 1:9 chromatographiert.

Ausbeute: 430 mg (48,7 % der Theorie)
$^1$H-NMR ($CDCl_3$): $\delta$ = 1,32 (d, 6H); 3,32 (sept., 1H); 3,61 (sept., 1H); 3,83 (s, 3H); 6,03 (dd, 1H); 7,0-7,2 (m, 4H); 7,28 (d, 1H); 7,77 (s, 1H) ppm.

Beispiel 5

Methyl-(E)-7-[2,6-diisopropyl-4-(4-fluorphenyl)-5-methoxyiminomethyl-pyrid-3-yl]-5-hydroxy-3-oxo-hept-6-enoat

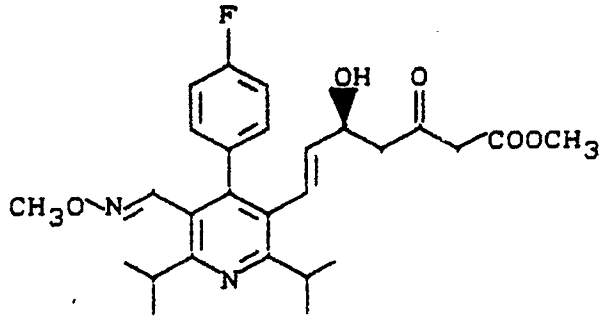

Unter Stickstoff tropft man zu einer Suspension von 67 mg (2,2 mmol) 80 %igem Natriumhydrid in 10 ml trockenem Tetrahydrofuran bei -5° C 0,18 ml (1,65 mmol) Acetessigsäuremethylester in 5 ml trockenem Tetrahydrofuran. Nach 15 min werden bei derselben Temperatur 1,01 ml (1,65 mmol) 15 %iges Butyllithium in n-Hexan zugetropft und 15 min nachgerührt. Anschließend werden 410 mg (1,1 mmol) der Verbindung aus Beispiel 4 gelöst in 10 ml trockenem Tetrahydrofuran gegeben und rührt 30 min bei -5° C nach. Die Reaktionslösung wird vorsichtig mit 0,3 ml Eisessig versetzt, mit 100 ml Wasser verdünnt und die Mischung dreimal mit je 100 ml Ether extrahiert.

Die vereinigten organischen Phasen werden zweimal mit gesättigter Natriumhydrogencarbonat-Lösung gewaschen, über Magnesiumsulfat getrocknet und im Vakuum eingeengt. Der Rückstand wird über Kieselgel filtriert (Lösungsmittel: Essigester/Petrolether 1:1).

Ausbeute: 490 mg (91,6 % der Theorie)

$^1$H-NMR ($CDCl_3$): $\delta$ = 1,25 (m, 6H); 2,47 (m, 2H); 3,29 (sept., 1H); 3,42 (s, 2H); 3,58 (sept. , 1H); 3,75 (s, 3H); 3,82 (s, 3H); 4,51 (m, 1H) 5,38 (dd, 1H); 6,36 (d, 1 H); 7,0-7,2 (m, 4H); 7,77 (s, 1H) ppm.

Beispiel 6

Methyl-erythro-(E)-7-[2,6-diisopropyl-4-(4-fluorphenyl)-5-methoxyiminomethyl-pyrid-3-yl]-3,5-dihydroxy-hept-6-enoat

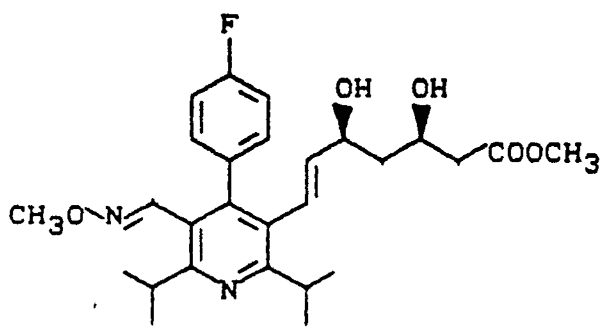

Zu einer Lösung von 470 mg (1 mmol) der Verbindung aus Beispiel 5 in 10 ml trockenem Tetrahydrofuran gibt man bei Raumtemperatur 1,2 ml (1,2 mmol) 1 M Triethylboran-Lösung in Tetrahydrofuran, leitet während 5 min Luft durch die Lösung und kühlt auf -30° C Innentemperatur ab. Es werden 46 mg (1,2 mmol) Natriumborhydrid und langsam 0,8 ml Methanol zugegeben, 30 min bei -30° C gerührt und dann mit einem Gemisch von 3 ml 30 %igem Wasserstoffperoxid und 10 ml Wasser versetzt. Die Temperatur läßt man dabei bis 0° C ansteigen und rührt noch 30 min nach. Die Mischung wird dreimal mit je 70 ml Essigester extrahiert, die vereinigten organischen Phasen je einmal mit gesättigter Natriumhydrogencarbonat-Lösung und gesättigter Natriumchlorid-Lösung gewaschen, über Magnesiumsulfat getrocknet und im Vakuum eingeengt. Der Rückstand wird an einer Säule (80 g Kieselgel 230-400 mesh,

Durchmesser 2,5 cm, mit Essigester/Petrolether 1:1) chromatographiert.

Ausbeute: 200 mg (41,1 % der Theorie)

$^1$H-NMR ($CDCl_3$): δ 1,25 (m, 12H); 1,43 (m, 2H); 2,42 (m, 2H); 3,32 (sept., 1H); 3,58 (m, 1H); 3,73 (s, 3H); 3,81 (s, 3H); 4,08 (m, 1H); 4,32 (m, 1H); 5,28 (dd, 1 H); 6,33 (d, 1H); 7,0-7,1 (m, 4H); 7,77 (s, 1H) ppm.

Beispiel 7

Natrium-erythro-(E)-7-[2,6-diisopropyl-4-(4-fluor-phenyl)-5-methoxyiminomethyl-pyrid-3-yl]-3,5-dihydroxy-hept-6-enoat

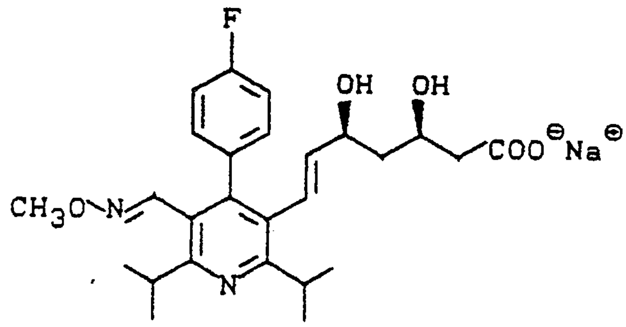

150 mg (0,3 mmol) der Verbindung aus Beispiel 6 werden in 10 ml Tetrahydrofuran gelöst und mit 3 ml 0,1 N Natronlauge versetzt. Nach 1 h wird das Tetrahydrofuran am Vakuum abgezogen und der wässrige Rückstand wird gefriergetrocknet.

Ausbeute: 143 mg (97 % der Theorie)

$^1$H-NMR ($CDCl_3$): δ = 0,89 (m, 1H); 1,22 (m, 12H); 1,32 (m, 1H); 1,27 (dd, 1H); 1,95 (dd, 1H); 3,31 (s, 3H); 3,38 (sept., 1H); 3,52 (sept., 1H); 4,03 (m, 1H); 4,92 (m, 1 H); 5,31 (dd, 1H); 6,18 (d, 1H); 7,0-7,3 (m, 4H); 7,78 (s, 1H) ppm.

Beispiel 8

trans-(E)-6-[2-(2,6-Diisopropyl-4-(4-fluorphenyl)-3-methoxyiminomethyl-pyrid-5-yl)-ethenyl]-3,4,5,6-tetrahydro-4-hydroxy-2H-pyran-2-on

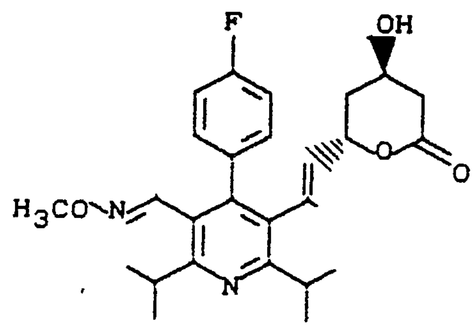

40 mg (0,08 mmol) der Verbindung aus Beispiel 6 werden in 10 ml Tetrahydrofuran gelöst und nach Zugabe von 0,8 ml (0,08 mmol) 0,1 N Natronlauge 1 h bei Raumtemperatur gerührt. Anschließend wird mit 10 ml Wasser verdünnt, mit 1 N Salzsäure auf pH 4,4 gestellt und mit Methylenchlorid mehrmals extrahiert. Die vereinigten organischen Phasen werden mit Natriumsulfat getrocknet und im Vakuum eingeengt. Der Rückstand wird in 20 ml absol. Toluol gelöst und nach Zugabe von 5 g Molekularsieb 4 Å über Nacht unter Rückfluß erhitzt. Anschließend wird vom Molekularsieb abfiltriert, eingeengt und über eine kurze Kieselgelsäule filtriert (Laufmittel Essigester/Petrolether 1:1).

Ausbeute: 21,1 mg (58,1 % der Theorie)

$^1$H-NMR ($CDCl_3$): δ b = 1,28 (m, 12H); 1,4-1,9 (m, 2H); 2,6 (m, 2H); 3,31 (sept., 1H); 3,57 (sept., 1H); 3,82 (s, 3H); 4,18 (m, 1H); 5,08 (m, 1H) 5,32 (dd, 1H); 6,42 (d, 1 H); 7,0-7,2 (m, 4H); 7,77 (s, 1H) ppm.

Beispiel 9

3-Benzyloxyiminomethyl-5-tert . -butyldimethylsilyloxymethyl-2,6-diisopropyl-4-(4-fluorphenyl)-pyridin

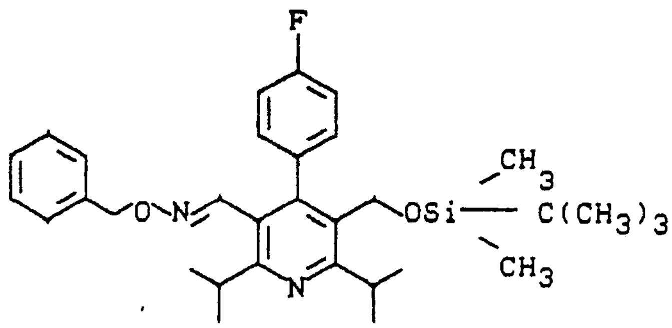

Analog Beispiel 1 erhält man aus 2,1 g (5 mmol) der Verbindung aus Beispiel VII und 922,5 mg (7,5 mmol) Benzylhydroxylamin Hydrochlorid die Titelbindung.

Ausbeute 1,55 g (57 % der Theorie)

$^1$H-NMR ($CDCl_3$): $\delta$ = 0,01 (s, 6H); 0,91 (s, 9H); 1,28 (d, 6H); 1,39 (d, 6H); 3,50 (m, 2H); 4,37 (s, 2H); 5,13 (s, 2H); 7,0-7,5 (m, 9H); 7,87 (s, 1H) ppm.

Beispiel 10

Methyl-erythro-(E)-7-[5-benzyloxyiminomethyl-2,6-diisopropyl-4-(4-fluorphenyl)-pyrid-3-yl]-3,5-dihydroxy-hept-6-enoat

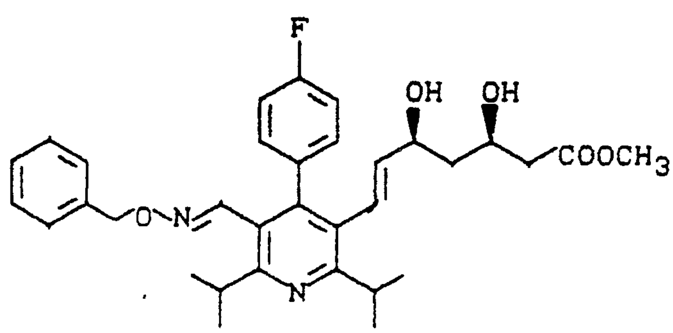

Aus der Verbindung aus Beispiel 9 wurde, in Analogie zu den Reaktionen aus den Beispielen 2-6, das Beispiel 10 hergestellt.

$^1$H-NMR ($CDCl_3$): $\delta$ = 1,26 (m, 12H); 1,42 (m, 2H); 2,43 (m, 2H); 3,31 (sept., 1H); 3,44 (sept., 1H); 3,72 (s, 3H); 4,08 (m, 1H); 4,30 (m, 1H); 5,05 (s, 2H); 5,26 (dd, 1H); 6,30 (d, 1H); 6,98 (m, 4H); 7,2-7,4 (m, 5H); 7,82 (s, 1H) ppm.

Beispiel 11

3-tert.-Butoxyiminomethyl-5-tert.-butyldimethylsilyloxymethyl-2,6-diisopropyl-4-(4-fluorphenyl)-pyridin

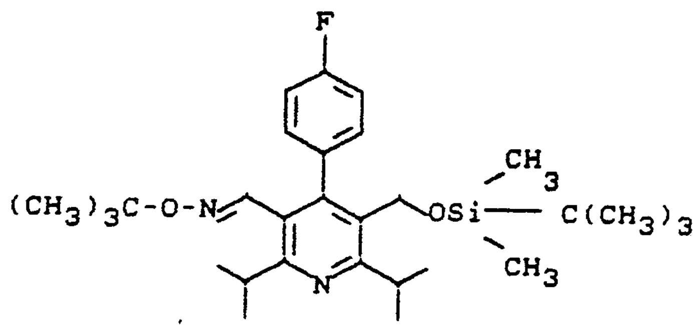

Analog Beispiel 1 erhält man aus 2,1 g (5 mmol) der Verbindung aus Beispiel VII und 941 mg (7,5 mmol) o-tert.-Butylhydroxylamin Hydrochlorid die Titelbindung.

Ausbeute 770 mg (30,8 % der Theorie)

$^1$H-NMR ($CDCl_3$): $\delta$ = 0,0 (s, 6H); 0,89 (s, 9H); 1,28 (s, 9H); 1,40 (m, 6H); 3,48 (sept., 1H); 3,63 (sept., 1H); 4,37 (s, 2H); 7,1-7,3 (m, 4H); 7,82 (s, 1H) ppm.

Beispiel 12

Methyl-erythro-(E)-7-[5-tert.-butyloxyiminomethyl-2,6-diisopropyl-4-(4-fluorphenyl)pyrid-3-yl]-3,5-dihydroxy-hept-6-enoat

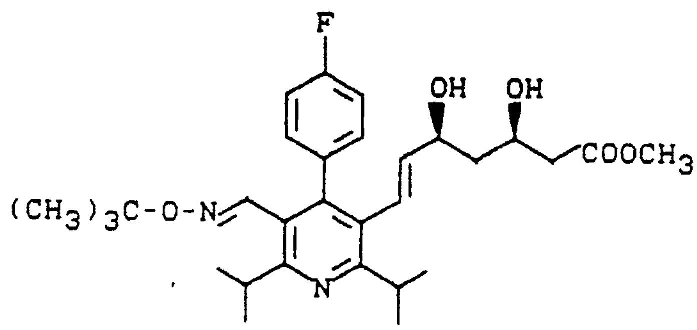

Aus der Verbindung aus Beispiel 11 wurde in Analogie zu den Reaktionen aus den Beispielen 2-6, das Beispiel 12 hergestellt.

$^1$H-NMR ($CDCl_3$): $\delta$ = 1,1-1,3 (m, 21H); 1,42 (m, 2H); 2,42 (m, 2H); 3,32 (sept. 1H); 3,53 (sept, 1H); 3,73 (s, 3H); 4,08 (m, 1H); 4,30 (m, 1H); 5,28 (dd, 1H) 6,31 (d, 1H); 7,0-7,1 (m, 4H); 7,78 (s, 1H) ppm.

**Ansprüche**

1. Imino-substituierte Pyridine der allgemeinen Formel

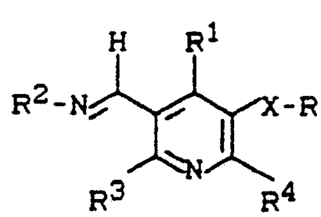

(I)

in welcher
$R^1$ für Aryl mit 6 bis 10 Kohlenstoffatomen steht, das gegebenenfalls bis zu 3-fach gleich oder verschieden durch Halogen, Hydroxy, Trifluormethyl, Trifluormethoxy, Nitro, Cyano, geradkettiges oder verzweigtes Alkyl oder Alkoxy mit jeweils bis zu 8 Kohlenstoffatomen oder durch Aryloxy mit 6 bis 10 Kohlenstoffatomen substituiert ist,
$R^2$ - für Aryl mit 6 bis 10 Kohlenstoffatomen steht, das gegebenenfalls bis zu 3-fach gleich oder verschieden durch geradkettiges oder verzweigtes Alkyl mit jeweils bis zu 8 Kohlenstoffatomen, Halogen, Cyano, Trifluormethyl, Trifluormethoxy oder Nitro substituiert ist, oder
- für geradkettiges oder verzweigtes Alkyl mit bis zu 10 Kohlenstoffatomen steht, das gege benenfalls durch Aryl mit 6 bis 10 Kohlenstoffatomen, Hydroxy oder Alkoxy mit bis zu 8 Kohlenstoffatomen substituiert ist, oder
- für eine Gruppe der Formel $-OR^5$ steht, worin
$R^5$ - Wasserstoff oder geradkettiges oder verzweigtes Alkyl mit bis zu 8 Kohlenstoffatomen bedeutet, das gegebenenfalls durch Aryl mit 6 bis 10 Kohlenstoffatomen substiutuiert ist, oder
- Aryl mit 6 bis 10 Kohlenstoffatomen bedeutet, das gegebenenfalls durch geradkettiges oder verzweigtes Alkyl oder Alkoxy mit jeweils bis zu 8 Kohlenstoffatomen, Halogen, Nitro, Cyano, Trifluormethyl oder Trifluormethoxy substituiert ist,
$R^3$ - für Cycloalkyl mit 3 bis 8 Kohlenstoffatomen steht, oder
- für Aryl mit 6 bis 10 Kohlenstoffatomen steht, das gegebenenfalls bis zu 3-fach gleich oder verschieden durch Halogen oder durch gerad kettiges oder verzweigtes Alkyl mit bis zu 8 Kohlenstoffatomen substituiert ist, oder
- für geradkettiges oder verzweigtes Alkyl mit bis zu 10 Kohlenstoffatomen steht, das gegebenenfalls durch eine Gruppe der Formel $-OR^5$ substituiert ist,
worin
$R^5$ die oben angegebene Bedeutung hat,
$R^4$ - für geradkettiges oder verzweigtes Alkyl mit bis zu 10 Kohlenstoffatomen steht, oder steht
X - für eine Gruppe der Formel $-CH_2-CH_2-$ oder $-CH=CH-$steht,
und
R - für eine Gruppe der Formel

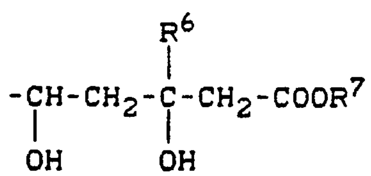 oder 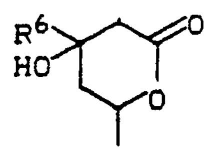 steht,

worin
$R^6$ - Wasserstoff oder geradkettiges oder verzweigtes Alkyl mit bis zu 10 Kohlenstoffatomen bedeutet
und
$R^7$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,
und deren Salze.
2. Imino-substituierte Pyridine der Formel I nach Anspruch 1,
worin
$R^1$ - für Phenyl steht, das gegebenenfalls bis zu 2-fach gleich oder verschieden durch Fluor, Chlor, Brom, Hydroxy, geradkettiges oder verzweigtes Alkyl oder Alkoxy mit jeweils bis zu 6 Kohlenstoffatomen oder durch Phenoxy substituiert ist,
$R^2$ - für Phenyl steht, das gegebenenfalls bis zu 2-fach gleich oder verschieden durch Fluor,
Chlor, Brom oder durch geradkettiges oder verzweigtes Alkyl mit bis zu 6 Kohlenstoffatomen substituiert ist, oder
- für geradkettiges oder verzweigtes Alkyl mit bis zu 8 Kohlenstoffatomen steht, das gegebenenfalls durch Phenyl oder Alkoxy mit bis zu 6 Kohlenstoffatomen substituiert ist, oder
- für eine Gruppe der Formel $-OR^5$ steht
worin

$R^5$ - Wasserstoff oder geradkettiges oder verzweigtes Alkyl mit bis zu 6 Kohlenstoffatomen bedeutet, das gegebenenfalls durch Phenyl substituiert ist,
oder
- Phenyl bedeutet, das gegebenenfalls durch geradkettiges oder verzweigtes Alkyl oder Alkoxy mit jeweils bis zu 6 Kohlenstoffatomen substituiert ist,
$R^3$ - für Cyclopropyl, Cyclobutyl, Cyclopentyl oder Cyclohexyl steht, oder
- für Phenyl steht, das gegebenenfalls bis zu 2-fach gleich oder verschieden durch Fluor, Chlor, Brom oder durch geradkettiges oder verzweigtes Alkyl mit bis zu 6 Kohlenstoffatomen substituiert ist, oder
- für geradkettiges oder verzweigtes Alkyl mit bis zu 8 Kohlenstoffatomen steht, das gegebenenfalls durch eine Gruppe der Formel $-OR^5$ substituiert ist
worin
$R^5$ die oben angegebene Bedeutung hat,
$R^4$ - für geradkettiges oder verzweigtes Alkyl mit bis zu 8 Kohlenstoffatomen steht, oder
- für Cyclopropyl, Cyclopentyl oder Cyclohexyl steht,
X - für eine Gruppe der Formel $-CH_2-CH_2-$ oder $-CH=CH-$steht
und
R - für eine Gruppe der Formel

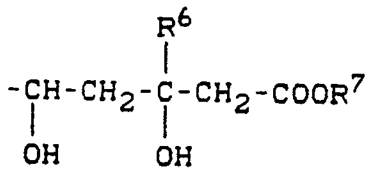 oder 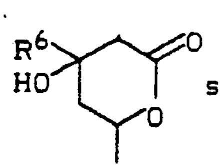 steht,

worin
$R^6$ - Wasserstoff oder geradkettiges oder verzweigtes Alkyl mit bis zu 8 Kohlenstoffatomen bedeutet
und
$R^7$ - Wasserstoff oder geradkettiges oder verzweigtes Alkyl mit bis zu 8 Kohlenstoffatomen oder Benzyl bedeutet, oder - Phenyl oder ein Kation bedeutet,
und deren Salze.
3. Imino-substituierte Pyridine der Formel I nach Anspruch 1,
worin
$R^1$ - für Phenyl steht, das gegebenenfalls durch Fluor, Chlor, geradkettiges oder verzweigtes Alkyl mit bis zu 4 Kohlenstoffatomen oder Phenoxy substituiert ist, oder
$R^2$ - für eine Gruppe der Formel $-OR^5$ steht worin
$R^5$ - Wasserstoff, Benzyl oder geradkettiges oder verzweigtes Alkyl mit bis zu 4 Kohlenstoffatomen bedeutet oder - Phenyl bedeutet,
$R^3$ - für Cyclopropyl oder Phenyl steht, oder
- für geradkettiges oder verzweigtes Alkyl mit bis zu 6 Kohlenstoffatomen steht, das gegebenenfalls durch eine Gruppe der Formel $-OR^5$ substituiert ist
worin
$R^5$-die oben angegebene Bedeutung hat,
$R^4$ - für geradkettiges oder verzweigtes Alkyl mit bis zu 6 Kohlenstoffatomen oder Cyclopropyl steht,
X - für eine Gruppe $-CH=CH-$ steht,
und
R - für eine Gruppe der Formel

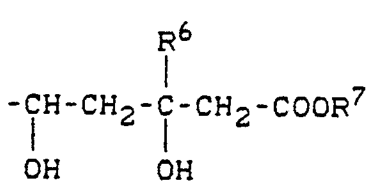 oder 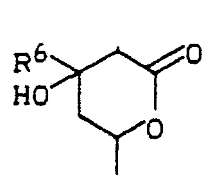 steht,

worin

$R^6$ - Wasserstoff, Methyl, Ethyl, Propyl, Isopropyl, Butyl, Isobutyl oder tert.Butyl bedeutet und

$R^7$ - Wasserstoff, Methyl, Ethyl, Propyl, Isopropyl, Butyl, Isobutyl, tert.Butyl oder Benzyl bedeutet, oder

- ein Natrium-, Kalium-, Calcium-, Magnesium-oder Ammoniumion bedeutet

und deren Salze

4. Imino-substituierte Pyridine nach den Ansprüchen 1 bis 3 zur Bekämpfung von Krankheiten.

5. Verfahren zur Herstellung von Imino-substituierten Pyridinen der allgemeinen Formel

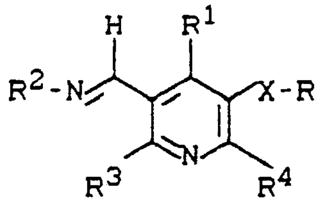

(I)

in welcher

$R^1$ für Aryl mit 6 bis 10 Kohlenstoffatomen steht, das gegebenenfalls bis zu 3-fach gleich oder verschieden durch Halogen, Hydroxy, Trifluormethyl, Trifluormethoxy, Nitro, Cyano, geradkettiges oder verzweigtes Alkyl oder Alkoxy mit jeweils bis zu 8 Kohlenstoffatomen oder durch Aryloxy mit 6 bis 10 Kohlenstoffatomen substituiert ist,

$R^2$ - für Aryl mit 6 bis 10 Kohlenstoffatomen steht, das gegebenenfalls bis zu 3-fach gleich oder verschieden durch geradkettiges oder verzweigtes Alkyl mit jeweils bis zu 8 Kohlenstoffatomen, Halogen, Cyano, Trifluormethyl, Trifluormethoxy oder Nitro substituiert ist,

oder

- für geradkettiges oder verzweigtes Alkyl mit bis zu 10 Kohlenstoffatomen steht, das gegebenenfalls durch Aryl mit 6 bis 10 Kohlenstoffatomen, Hydroxy oder Alkoxy mit bis zu 8 Kohlenstoffatomen substituiert ist,

oder

- für eine Gruppe der Formel $-OR^5$ steht,

worin

$R^5$ - Wasserstoff oder geradkettiges oder verzweigtes Alkyl mit bis zu 8 Kohlenstoffatomen bedeutet, das gegebenenfalls durch Aryl mit 6 bis 10 Kohlenstoffatomen substiutuiert ist,

oder

- Aryl mit 6 bis 10 Kohlenstoffatomen bedeutet, das gegebenenfalls durch geradkettiges oder verzweigtes Alkyl oder Alkoxy mit jeweils bis zu 8 Kohlenstoff atomen, Halogen, Nitro, Cyano, Trifluormethyl oder Trifluormethoxy substituiert ist,

$R^3$ - für Cycloalkyl mit 3 bis 8 Kohlenstoffatomen steht, oder

- für Aryl mit 6 bis 10 Kohlenstoffatomen steht, das gegebenenfalls bis zu 3-fach gleich oder verschieden durch Halogen oder durch geradkettiges oder verzweigtes Alkyl mit bis zu 8 Kohlenstoffatomen substituiert ist, oder

- für geradkettiges oder verzweigtes Alkyl mit bis zu 10 Kohlenstoffatomen steht, das gegebenenfalls durch eine Gruppe der Formel $-OR^5$ substituiert ist,

worin

$R^5$ die oben angegebene Bedeutung hat,

$R^4$ - für geradkettiges oder verzweigtes Alkyl mit bis zu 10 Kohlenstoffatomen steht, oder

- für Cycloalkyl mit 3 bis 8 Kohlenstoffatomen steht

X - für eine Gruppe der Formel $-CH_2-CH_2-$ oder $-CH=CH-$steht,

und

R - für eine Gruppe der Formel

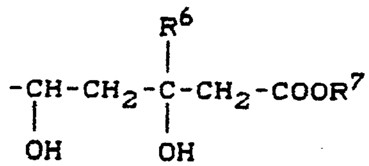 oder 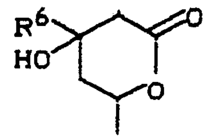 steht,

worin
$R^6$ - Wasserstoff oder geradkettiges oder verzweigtes Alkyl mit bis zu 10 Kohlenstoffatomen bedeutet
und
$R^7$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,
und deren Salze, dadurch gekennzeichnet, daß man Ketone der allgemeinen Formel

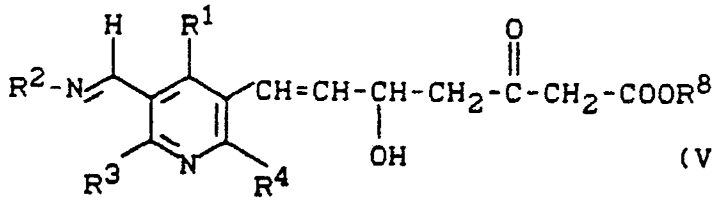 (VIII)

in welcher
$R^1$, $R^2$, $R^3$, $R^4$, X und R die oben angegebene Bedeutung haben
und
$R^8$ - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X = -CH=CH-) nach üblichen Methoden hydriert,
und gegebenenfalls Isomeren trennt.

6. Arzneimittel enthaltend mindestens ein Imino-substituiertes Pyridin nach Anspruch 1.

7. Verfahren zur Herstellung eines Arzneimittels nach Anspruch 6, dadurch gekennzeichnet, daß man Iminosubstituierte Pyridine nach Anspruch 1 gegebenenfalls mit Hilfe von üblichen Hilfs- und Trägerstoffen in eine geeignete Applikationsform bringt.

8. Verwendung von Imino-substituierten Pyridinen nach Anspruch 1 zur Herstellung von Arzneimitteln.

9. Verwendung von Imino-substituierten Pyridinen nach Anspruch 1 zur Herstellung von Arzneimitteln zur Behandlung von Hyperlipoproteinämie, Lipoproteinämie, Atherosklerose und zur Senkung des Cholerestingehaltes im Blut.

10. Verwendung von Imino-substituierten Pyridinen nach Anspruch 1 zur Bekämpfung von Krankheiten.

11. Ketone der allgemeinen Formel

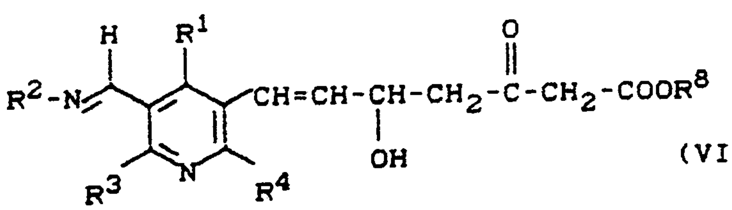 (VIII)

in welcher

$R^1$ für Aryl mit 6 bis 10 Kohlenstoffatomen steht, das gegebenenfalls bis zu 3-fach gleich oder verschieden durch Halogen, Hydroxy, Trifluormethyl, Trifluormethoxy, Nitro, Cyano, geradkettiges oder verzweigtes Alkyl oder Alkoxy mit jeweils bis zu 8 Kohlenstoffatomen oder durch Aryloxy mit 6 bis 10 Kohlenstoffatomen substituiert ist,

$R^2$ - für Aryl mit 6 bis 10 Kohlenstoffatomen steht, das gegebenenfalls bis zu 3-fach gleich oder verschieden durch geradkettiges oder verzweigtes Alkyl mit jeweils bis zu 8 Kohlenstoffatomen, Halogen, Cyano, Trifluormethyl, Trifluormethoxy oder Nitro substituiert ist, oder

- für geradkettiges oder verzweigtes Alkyl mit bis zu 10 Kohlenstoffatomen steht, das gegebenenfalls durch Aryl mit 6 bis 10 Kohlenstoffatomen, Hydroxy oder Alkoxy mit bis zu 8 Kohlenstoffatomen substituiert ist, oder

- für eine Gruppe der Formel $-OR^5$ steht, worin

$R^5$ - Wasserstoff oder geradkettiges oder verzweigtes Alkyl mit bis zu 8 Kohlenstoffatomen bedeutet, das gegebenenfalls durch Aryl mit 6 bis 10 Kohlenstoffatomen substituiert ist,

oder

- Aryl mit 6 bis 10 Kohlenstoffatomen bedeutet, das gegebenenfalls durch geradkettiges oder verzweigtes Alkyl oder Alkoxy mit jeweils bis zu 8 Kohlenstoffatomen, Halogen, Nitro, Cyano, Trifluormethyl oder Trifluormethoxy substituiert ist,

$R^3$ - für Cycloalkyl mit 3 bis 8 Kohlenstoffatomen steht, oder

- für Aryl mit 6 bis 10 Kohlenstoffatomen steht, das gegebenenfalls bis zu 3-fach gleich oder verschieden durch Halogen oder durch geradkettiges oder verzweigtes Alkyl mit bis zu 8 Kohlenstoffatomen substituiert ist, oder

- für geradkettiges oder verzweigtes Alkyl mit bis zu 10 Kohlenstoffatomen steht, das gegebenenfalls durch eine Gruppe der Formel $-OR^5$ substituiert ist,

worin

$R^5$ die oben angegebene Bedeutung hat,

$R^4$ - für geradkettiges oder verzweigtes Alkyl mit bis zu 10 Kohlenstoffatomen steht, oder

- für Cycloalkyl mit 3 bis 8 Kohlenstoffatomen steht

X - für eine Gruppe der Formel $-CH_2-CH_2-$ oder $-CH=CH-$ steht,

und

R - für eine Gruppe der Formel

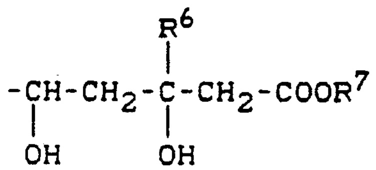 oder 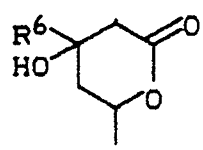 steht,

worin

$R^6$ - Wasserstoff oder geradkettiges oder verzweigtes Alkyl mit bis zu 10 Kohlenstoffatomen bedeutet

und

$R^7$ - Wasserstoff oder geradkettiges oder verzweigtes Alkyl mit bis zu 10 Kohlenstoffatomen bedeutet, das durch Phenyl substituiert sein kann, oder

- Aryl mit 6 bis 10 Kohlenstoffatomen oder ein Kation bedeutet.

12. Verfahren zur Herstellung von Ketonen der allgemeinen Formel

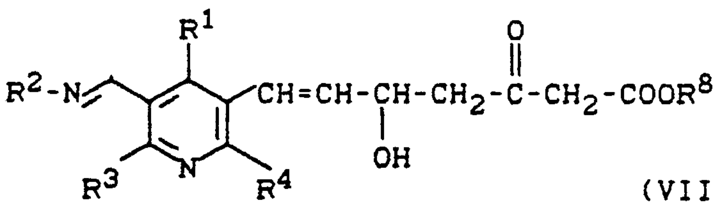

(VIII)

in welcher

$R^1$ für Aryl mit 6 und $R^8$ bis 10 Kohlenstoffatomen steht, das gegebenenfalls bis zu 3-fach gleich oder verschieden durch Halogen, Hydroxy, Trifluormethyl, Trifluormethoxy, Nitro, Cyano, geradkettiges oder verzweigtes Alkyl oder Alkoxy mit jeweils bis zu 8 Kohlenstoffatomen oder durch Aryloxy mit 6 bis 10 Kohlenstoffatomen substituiert ist,

$R^2$ - für Aryl mit 6 bis 10 Kohlenstoffatomen steht, das gegebenenfalls bis zu 3-fach gleich oder verschieden durch geradkettiges oder verzweigtes Alkyl mit jeweils bis zu 8 Kohlenstoffatomen, Halogen, Cyano, Trifluormethyl, Trifluormethoxy oder Nitro substituiert ist, oder

- für geradkettiges oder verzweigtes Alkyl mit bis zu 10 Kohlenstoffatomen steht, das gegebenenfalls durch Aryl mit 6 bis 10 Kohlenstoffatomen, Hydroxy oder Alkoxy mit bis zu 8 Kohlenstoffatomen substituiert ist, oder

- für eine Gruppe der Formel $-OR^5$ steht,

worin

$R^5$ - Wasserstoff oder geradkettiges oder verzweigtes Alkyl mit bis zu 8 Kohlenstoffatomen bedeutet, das gegebenenfalls durch Aryl mit 6 bis 10 Kohlenstoffatomen substituiert ist

oder

- Aryl mit 6 bis 10 Kohlenstoffatomen bedeutet, das gegebenenfalls durch geradkettiges oder verzweigtes Alkyl oder Alkoxy mit jeweils bis zu 8 Kohlenstoffatomen, Halogen, Nitro, Cyano, Trifluormethyl oder Trifluormethoxy substituiert ist,

$R^3$ - für Cycloalkyl mit 3 bis 8 Kohlenstoffatomen steht, oder

- für Aryl mit 6 bis 10 Kohlenstoffatomen steht, das gegebenenfalls bis zu 3-fach gleich oder verschieden durch Halogen oder durch geradkettiges oder verzweigtes Alkyl mit bis zu 8 Kohlenstoffatomen substituiert ist, oder

- für geradkettiges oder verzweigtes Alkyl mit bis zu 10 Kohlenstoffatomen steht, das gegebenenfalls durch eine Gruppe der Formel $-OR^5$ substituiert ist,

worin

$R^5$ die oben angegebene Bedeutung hat,

$R^4$ - für geradkettiges oder verzweigtes Alkyl mit bis zu 10 Kohlenstoffatomen steht, oder

- für Cycloalkyl mit 3 bis 8 Kohlenstoffatomen steht

X - für eine Gruppe der Formel $-CH_2-CH_2-$ oder $-CH=CH-$steht,

und

R - für eine Gruppe der Formel

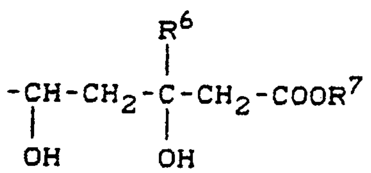

oder

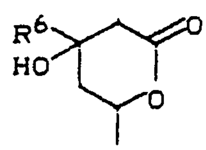

steht,

worin

$R^6$ - Wasserstoff oder geradkettiges oder verzweigtes Alkyl mit bis zu 10 Kohlenstoffatomen bedeutet

und

$R^7$ - Wasserstoff oder geradkettiges oder verzweigtes Alkyl mit bis zu 10 Kohlenstoffatomen bedeutet, das durch Phenyl substituiert sein kann, oder

- Aryl mit 6 bis 10 Kohlenstoffatomen oder ein Kation bedeutet,

dadurch gekennzeichnet ist, daß man

Aldehyde der allgemeinen Formel

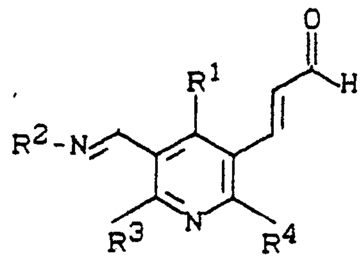

(IX)

in welcher
$R^1$, $R^2$, $R^3$, und $R^4$ die oben angegebene Bedeutung haben,
in inerten Lösemitteln mit Acetessigester der allgemeinen Formel

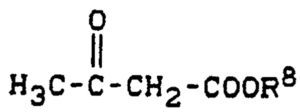

(X)

in welcher
$R^8$ die oben angegebene Bedeutung hat,
in Anwesenheit von Basen umsetzt.